# EUROPEAN PATENT APPLICATION

(11) **EP 2 949 324 A1**
(43) Date of publication of application: **02.12.2015**
(21) Application number: 14382190.8
(22) Date of filing: 27.05.2014
(51) Int. Cl.: A61K 31/22, A61K 31/366, A61K 31/40, A61K 31/404, A61K 31/4418, A61K 31/47, A61K 31/505, A61K 45/06, A61P 9/10

(54) **Prevention and/or treatment of ischemia/reperfusion injury**

(71) Applicant: Consorci Institut Catala de Ciencies Cardiovasculars, 08025 Barcelona (ES)
(72) Inventor: BADIMON MAESTRO, Lina, E-08025 Barcelona (ES); VILAHUR GARCIA, Gemma, E-08025 Barcelona (ES)
(74) Representative: ABG Patentes, S.L.

(57) **Abstract**

The present invention relates to compounds for their use in the prevention and/or treatment of ischemia/reperfusion injury of a tissue or an organ in a subject, wherein said compounds are statins, statin derivatives, agents inhibiting RhoA translocation to the cell membrane, agents increasing PKC_{ε} activity or any combination thereof, administered to the subject in need thereof prior to reperfusion.

SEQUENCE LISTING

<110>  Consorci Institut Català de Ciències Cardiovasculars

<120>  PREVENTION AND/OR TREATMENT OF ISCHEMIA/REPERFUSION INJURY

<130>  P10675EP00

<160>  2

<170>  PatentIn version 3.5

<210>  1
<211>  232
<212>  PRT
<213>  Artificial Sequence

<220>
<223>  BA-210

<400>  1

Met Ser Ala Tyr Ser Asn Thr Tyr Gln Glu Phe Thr Asn Ile Asp Gln
1               5                   10                  15

Ala Lys Ala Trp Gly Asn Ala Gln Tyr Lys Lys Tyr Gly Leu Ser Lys
            20                  25                  30

Ser Glu Lys Glu Ala Ile Val Ser Tyr Thr Lys Ser Ala Ser Glu Ile
        35                  40                  45

Asn Gly Lys Leu Arg Gln Asn Lys Gly Val Ile Asn Gly Phe Pro Ser
    50                  55                  60

Asn Leu Ile Lys Gln Val Glu Leu Leu Asp Lys Ser Phe Asn Lys Met
65                  70                  75                  80

Lys Thr Pro Glu Asn Ile Met Leu Phe Arg Gly Asp Asp Pro Ala Tyr
                85                  90                  95

Leu Gly Thr Glu Phe Gln Asn Thr Leu Leu Asn Ser Asn Gly Thr Ile
            100                 105                 110

Asn Lys Thr Ala Phe Glu Lys Ala Lys Ala Lys Phe Leu Asn Lys Asp
        115                 120                 125

Arg Leu Glu Tyr Gly Tyr Ile Ser Thr Ser Leu Met Asn Val Ser Gln
    130                 135                 140

Phe Ala Gly Arg Pro Ile Ile Thr Lys Phe Lys Val Ala Lys Gly Ser
145                 150                 155                 160

Lys Ala Gly Tyr Ile Asp Pro Ile Ser Ala Phe Ala Gly Gln Leu Glu

                165                 170                 175

Met Leu Leu Pro Arg His Ser Thr Tyr His Ile Asp Asp Met Arg Leu
            180                 185                 190

Ser Ser Asp Gly Lys Gln Ile Ile Ile Thr Ala Thr Met Met Gly Thr
        195                 200                 205

Ala Ile Asn Pro Lys Glu Phe Val Met Asn Pro Ala Asn Ala Gln Gly
    210                 215                 220

Arg His Thr Pro Gly Thr Arg Leu
225                 230


<210>  2
<211>  16
<212>  DNA
<213>  Artificial Sequence

<220>
<223>  RhoA antisense oligonucleotide

<400>  2
tcctgatggc agccat                                                    16

## Description

### FIELD OF THE INVENTION

The present invention relates, in general, to the prevention and/or treatment of ischemia/reperfusion injury; particularly, to the use of a statin or a derivative thereof, an agent inhibiting RhoA translocation to the cell membrane, an agent increasing PKCε activity, or any combination thereof, in the prevention and/or treatment of ischemia/reperfusion injury.

### BACKGROUND OF THE INVENTION

Reperfusion of ischemic tissues is often associated with microvascular dysfunction that is manifested as impaired endothelium-dependent dilation in arterioles, enhanced fluid filtration and leukocyte plugging in capillaries, and the trafficking of leukocytes and plasma protein extravasation in postcapillary venules. Activated endothelial cells in all segments of the microcirculation produce more oxygen radicals, but less nitric oxide, in the initial period following reperfusion. The resulting imbalance between superoxide and nitric oxide in endothelial cells leads to the production and release of inflammatory mediators (e.g. platelet-activating factor, tumor necrosis factor) and enhances the biosynthesis of adhesion molecules that mediate leukocyte-endothelial cell adhesion. Some of the known risk factors for cardiovascular disease (hypercholesterolemia, hypertension and diabetes) appear to exaggerate many of the microvascular alterations elicited by ischemia and reperfusion. The inflammatory mediators released as a consequence of reperfusion also appear to activate endothelial cells in remote organs that are not exposed to the initial ischemic insult. This distant response to ischemia/reperfusion can result in leukocyte-dependent microvascular injury that is characteristic of the multiple organ dysfunction syndrome.

Adaptational responses to ischemia/reperfusion injury have been demonstrated that allow for protection of briefly ischemic tissues against the harmful effects of subsequent, prolonged ischemia, a phenomenon called ischemic preconditioning. There are two temporally and mechanistically distinct types of protection afforded by this adaptational response, i.e. acute and delayed preconditioning. The factors (e.g. protein kinase C activation) that initiate the acute and delayed preconditioning responses appear to be similar; however the protective effects of acute preconditioning are protein synthesis-independent, while the effects of delayed preconditioning require protein synthesis.

During ischemia, there is loss of ATP and reduction in its synthesis, leading to an accumulation of the purine precursor hypoxanthine. Meanwhile, the enzyme that degrades hypoxanthine, xanthine dehydrogenase, is converted to xanthine oxidase, which transfers an electron onto O₂, creating an oxygen radical. This conversion is caused by calcium-dependent proteinases, whose activity is promoted by the increase in cytosolic calcium that occurs with ischemia. In addition, glutathione levels are decreased. This endogenous scavenger maintains cell membrane integrity. The role of nitric oxide (NO) in ischemia/reperfusion is complex. The effect appears to be time-dependent and/or enzyme-dependent wherein there is an early loss of NO (during ischemia) followed by subsequent excessive production (late reperfusion). The inflammatory response that occurs on reperfusion is a coordinated effort between the circulating leukocytes (mainly neutrophils) and the venular endothelium as well as the resident macrophages and mast cells. The reintroduction of O₂ results in a respiratory burst phenomenon with the production of toxic oxygen-derived free radicals, such as superoxide anion (O₂⁻), H₂O₂ and hydroxyl radical (OH^{•}). This generation of oxidants in excess of the capacity of the endogenous scavenging systems results in complement activation, production of leukotriene (LT)B4 and platelet activating factor (PAF), mobilization of P-selectin from the Weibel-Palade bodies, as well as synthesis of E-selectin and intracellular adhesion molecule (ICAM)-1 through the activation of nuclear transcription factors (eg, NFκB). There is also a change in the ionic composition of the cytoplasm with increased calcium. For example, in cardiac myocytes there is uncoupling of oxidative phosphorylation and tension generation due to energy wasting mechanisms, such as increased activation of calcium transport at the inner mitochondrial membrane and sarcoplasmic reticulum. This creates a vicious cycle where calcium causes an increase in xanthine oxidase, promoting the production of oxygen radicals, resulting in further calcium influx. The outpouring of cytokines, PAF and LTB4 promotes leukocyte recruitment leading to local and eventually systemic inflammation.

During ischemia there is a decrease in intracellular pH, which activates the Na⁺/H⁺ exchange system with extrusion of H⁺ from the cell in exchange for Na⁺. This leads to an increase in intracellular sodium, which continues during reperfusion. The normal Na⁺/K⁺ ATPase system, which extrudes sodium, is inhibited in this acid medium. This results in activation of the Na⁺/Ca²⁺ exchanger, leading to a buildup of intracellular calcium and eventually cell death. This is the rationale for the use of Na⁺/H⁺ exchange inhibitors. Cariporide (Aventis) is one compound that has been extensively studied, including in a recent clinical trial in which it demonstrated an attenuation of reperfusion injury characterized by improved left ventricular (LV) dysfunction. SM-20550 (Sumitomo), another Na⁺/H⁺-exchange inhibitor, was compared with nicorandil (Chugai Pharma), a K⁺ channel opener with nitrate-like activity. Both reduced infarct size in a dose-dependent manner when given pre-ischemia but only SM-20550 was beneficial when given post-ischemia.

Adenosine is an autocoid released by neutrophils, endothelium and myocytes, and is currently used in the treatment of cardiac arrhythmias. Its effects are mediated by specific tissue-dependent receptors. In a canine hepatic ischemia/reperfusion model, adenosine given prior to reperfusion attenuated the rise in aspartate aminotransferase (AST) and alanine transaminase (ALT), restored hepatic glutathione stores and inhibited oxidative damage to proteins resulting in inhibition of neutrophil accumulation and superoxide radical production. Using a canine cardiac ischemia model, CGS-21680 (Novartis), an adenosine A2A receptor analog, infused during reperfusion, upregulated Bcl-2 levels and downregulated Bax, reduced neutrophil accumulation in the perinecrotic myocardium and most importantly was not associated with bradycardia, unlike adenosine.

An increase in angiotensin (Ang) II is deleterious in the setting of ischemia/reperfusion. At pathophysiological levels, Ang II induces myocardial necrosis, promotes cardiac hypertrophy, positive inotropism and increases cardiac levels of norepinephrine, resulting in increased arrhythmogenicity and coronary vasoconstriction. Angiotensin converting enzyme (ACE) inhibitors have demonstrated significant clinical benefit, but in experimental models they have not been as effective as expected in attenuating reperfusion injury, due to the presence of ACE-independent enzymes, such as heart chymase, that convert Ang I to Ang II.

Endothelin, which is synthesized by the endothelium, is the most potent vasoconstrictor known and is responsible for the no reflow phenomenon, and activation and accumulation of neutrophils, as well as increased intracellular calcium concentrations. Selective antagonists to the two endothelin receptors have been developed and studied. Rats treated just prior to reperfusion with BQ-123 (ETA-selective; Banyu Pharmaceutical) or BQ-788 (ETB-selective; Banyu Pharmaceutical) had significantly better cardiac function and myocardial relaxation at 1 h, but the difference compared to placebo-treated animals was no longer evident at 24 h.

Experimental studies in isolated perfused mouse hearts and rats fed a normocholesterolemic diet have demonstrated the capability of statins to exert cardioprotection in the setting of ischemia/reperfusion, either when administered prior induction of ischemia (chronic and/or early deliver before induction of infarction) or after myocardial infarction (MI)-induction (Bell RM et al. 2003 J Am Coll Cardiol 41: 508-515; Vilahur G et al. 2009 Atherosclerosis 206: 95-101; Scalia R et al. 2001 Circulation 103: 2598-2603; Li XD et al. 2013 Int J Cardiol 167: 2657-2666). However, the optimization of the time of use and loading dose of statin during ischemia and prior reperfusion remains to be investigated.

Effective therapies to reduce or prevent ischemia/reperfusion injury have proven elusive. Despite an improved understanding of the pathophysiology of this process and encouraging preclinical trials of multiple agents, most of the clinical trials to prevent reperfusion injury have been disappointing. Therefore, in view of the state of the art, there is still a need to develop strategies to prevent damages caused by ischemia/reperfusion.

### SUMMARY OF THE INVENTION

The authors of the present invention have found that intravenous administration of a statin derivative (e.g., a beta-hydroxy acid derivative of simvastatin) during ischemia prior to reperfusion (before opening of an occluded artery in a pig model of closed-chest coronary ballon occlusion/reperfucion) induces heart protection by attenuating tissue and functional determinants of reperfusion injury despite co-morbid conditions, thus limiting reperfusion-related myocardial damage. Additionally, maintenance of the treatment with a statin (e.g., oral simvastatin) after reperfusion favors cardiac healing post-myocardial infarction.

Effectively, as it is shown in Example 1, intravenous infusion of a beta-hydroxy acid derivative of simvastatin during ischemia and prior to reperfusion in a pig model of closed-chest coronary balloon occlusion/reperfusion reduced coronary and cardiac oxidative DNA-damage, diminished neutrophils infiltration at the site of ischemia, preserved mitochondrial membrane potential and reduced apoptosis in the ischemic myocardium (lower mRNA levels of Fas, casp8, p53, and casp3 and mitochondrial-p-Bcl2, reduced TUNEL and active caspase-3). Further, this treatment regime attenuated reperfusion-related arrhythmias and stunning leading to a 40% increased salvage myocardium. Three weeks post-MI (myocardial infarction) simvastatin-treated animals showed P-PKCε increase, lower intramyocardial lipotoxicity, TβRII/Smad2/3 signaling restoration and subsequent myofibroblast differentiation and collagen-fibril formation in the evolving scar. Simvastatin suppressed cardiac RhoA mobilization and triggered Akt/eNOS signaling. Therefore, although the inventors do not wish to be bound by any theory, it appears that the inhibition of the 3-hydroxy-3-methyl-glutaryl-coenzyme A (HMG-CoA) reductase enzyme during ischemia and prior to reperfusion limits reperfusion injury, and that prolonged treatment with simvastatin thereafter improves cardiac healing post-MI. This therapeutic approach could be easily translated to patient treatment just before primary percutaneous coronary intervention (PCI) or surgical revascularization.

Therefore, in an aspect, the invention relates to a compound selected from the group consisting of: a) a statin or a derivative thereof, b) an agent inhibiting RhoA translocation to the cell membrane, c) an agent increasing PKCε activity, or any combination of a)-c), for use in the prevention and/or treatment of ischemia/reperfusion injury in a subject, wherein said compound is administered to said subject prior to reperfusion. Alternatively, the invention relates to the use of a compound selected from the group consisting of: a) a statin or a derivative thereof, b) an agent inhibiting RhoA translocation to the cell membrane, c) an agent increasing PKCε activity, or any combination of a)-c), in the manufacture of a medicament for the prevention and/or treatment of ischemia/reperfusion injury in a subject, wherein said medicament is administered to said subject prior to reperfusion.

In another aspect, the invention relates to a method for the prevention and/or treatment of ischemia/reperfusion injury in a subject in need thereof, which comprises administering to said subject, prior to reperfusion, a therapeutically effective amount of a compound selected from the group consisting of: a) a statin or a derivative thereof, b) an agent inhibiting RhoA translocation to the cell membrane, c) an agent increasing PKCε activity, and d) any combination of a)-c).

In another aspect, the invention relates to a kit comprising:
1) a first compound selected from the group consisting of: a) a statin or a derivative thereof, b) an agent inhibiting RhoA translocation to the cell membrane, c) an agent increasing PKCε activity, and d) any combination of a)-c), and
2) a second compound selected from the group consisting of: a) a statin or a derivative thereof, b) an agent inhibiting RhoA translocation to the cell membrane, c) an agent increasing PKCε activity, and d) any combination of a)-c).

In another aspect, the invention relates to the use of the above kit in the prevention and/or treatment of ischemia/reperfusion injury, wherein the first compound is intended to be administered prior to reperfusion and wherein the second compound is intended to be administered after reperfusion. Alternatively expressed, the invention relates to the above kit for use in the prevention and/or treatment of ischemia/reperfusion injury, wherein the first compound is intended to be administered prior to reperfusion and wherein the second compound is intended to be administered after reperfusion.

### BRIEF DESCRIPTION OF THE DRAWINGS

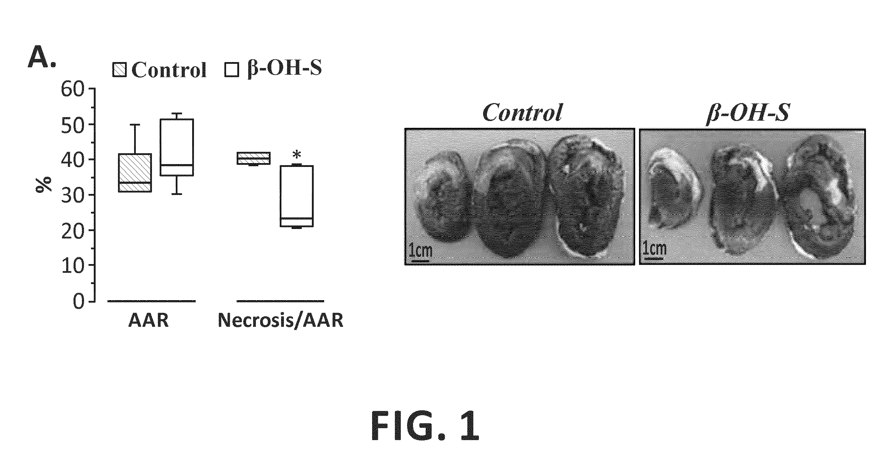

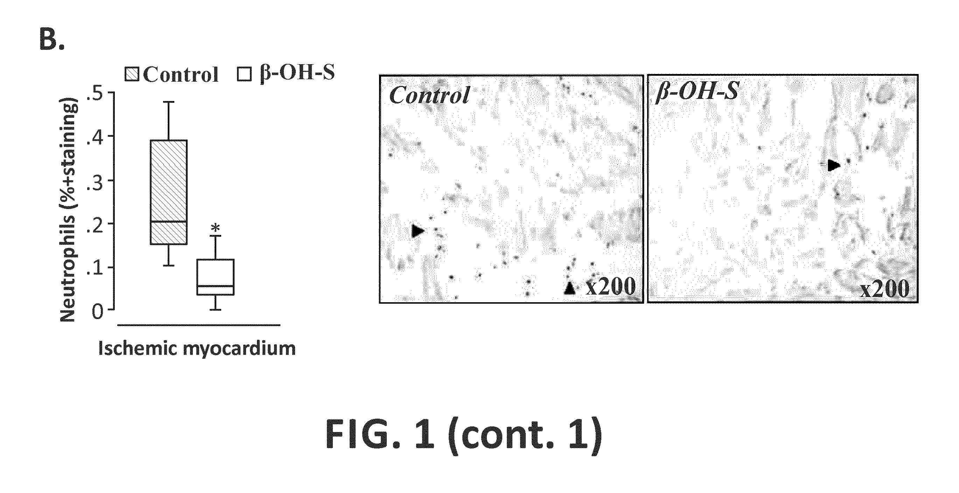

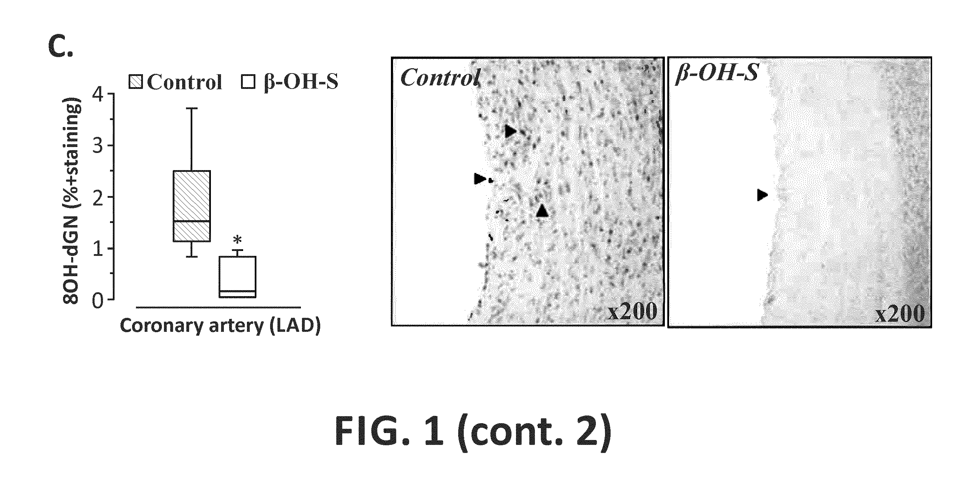

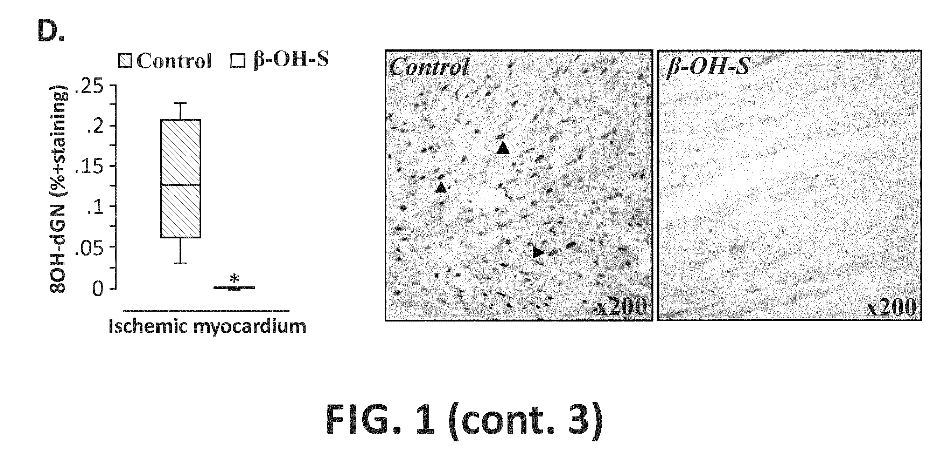

**Figure 1** shows the protective effect of intravenous (i.v.) beta-hydroxy acid derivative of simvastatin (β-OH-S) administration, prior to reperfusion, against ischemia/reperfusion-injury in a pig model of closed-chest coronary balloon occlusion/reperfusion. (A) Infarct size, (B) neutrophils myocardial recruitment, (C) oxidative DNA-damage in the coronary artery and (D) ischemic myocardium 2.5 h post-reperfusion. *p<0.05 *vs* vehicle/control animals. Black arrows depict neutrophils or positive staining.

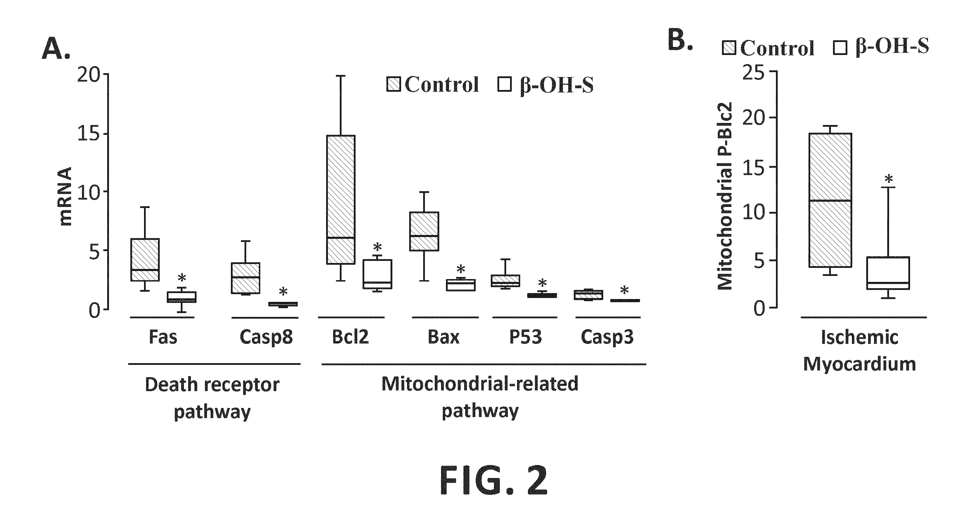

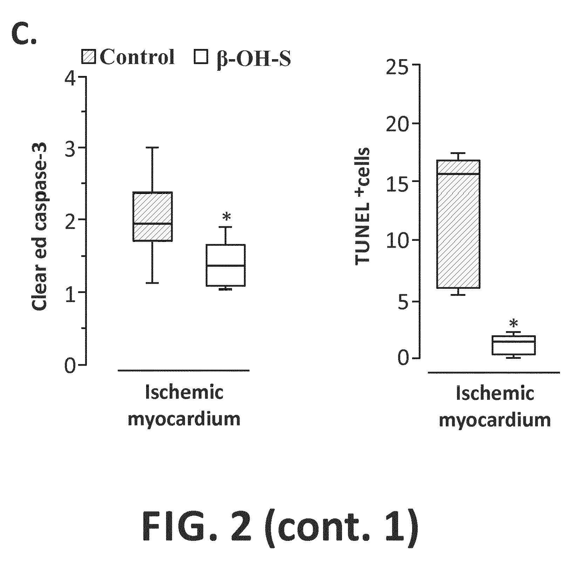

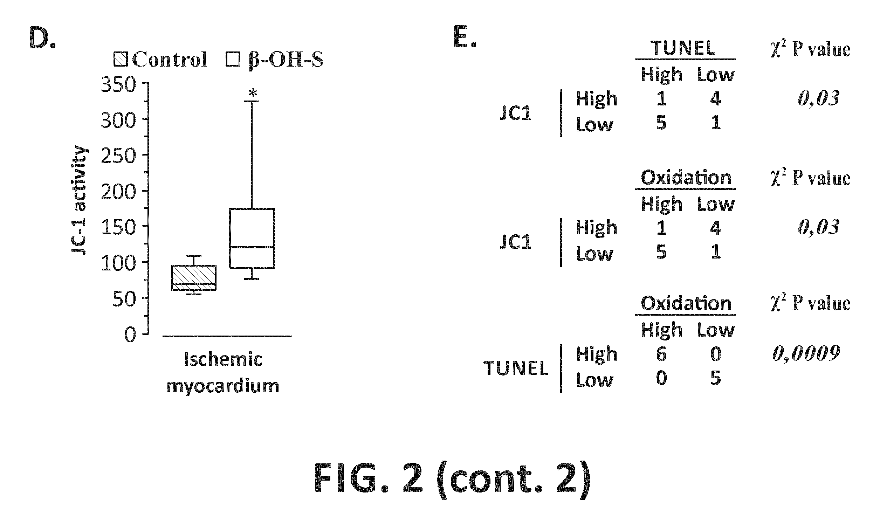

**Figure 2** shows the protective effect of i.v. β-OH-S administration, prior to reperfusion, on myocardial cell preservation in a pig model of closed-chest coronary balloon occlusion/reperfusion. (A) Gene expression of apoptosis-related markers. (B) Mitochondrial p-Bcl2 expression. (C) Caspase-3 activation. (D) Myocardial mitochondrial membrane potential. (E) Contengy tables to assess the potential association between oxidative damage, apoptosis and membrane potential. *p<0.05 *vs* vehicle/control animals.

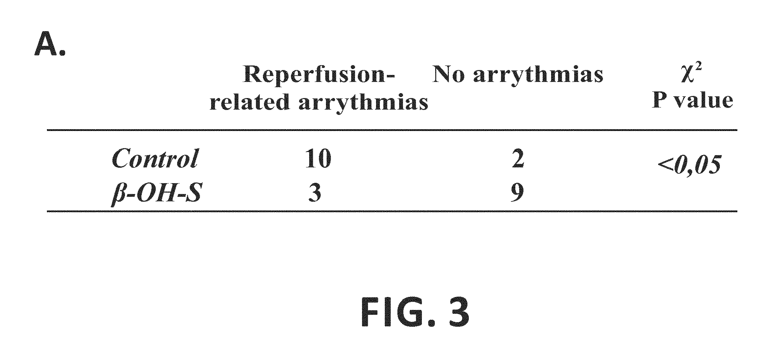

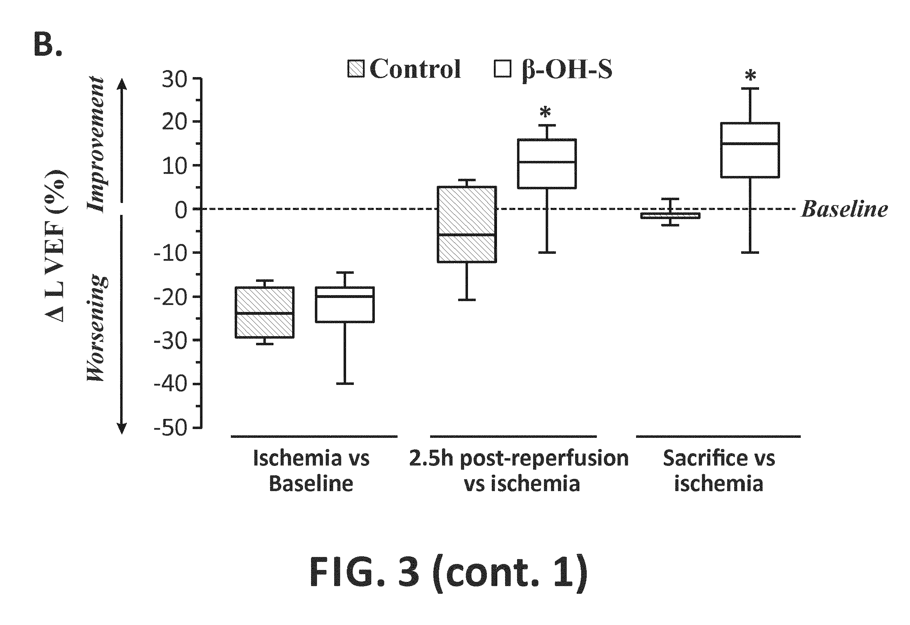

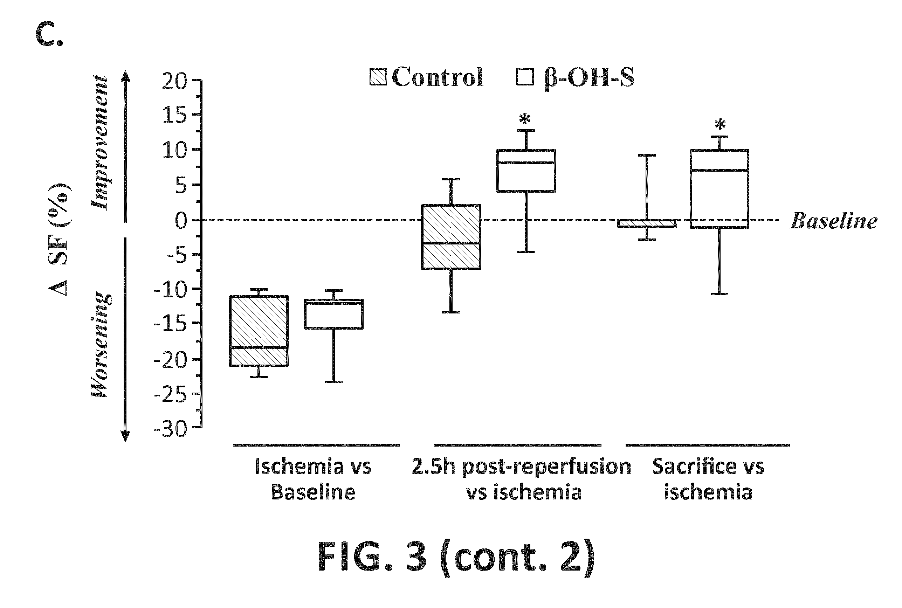

**Figure 3** shows the effect of i.v. β-OH-S administration, prior to reperfusion, on cardiac performance in a pig model of closed-chest coronary balloon occlusion/reperfusion. (A) Reperfusion-related arrhythmias, (B) left ventricle ejection fraction (LVEF) and (C) shortening fraction (SF). *p<0.05 *vs* vehicle/control.

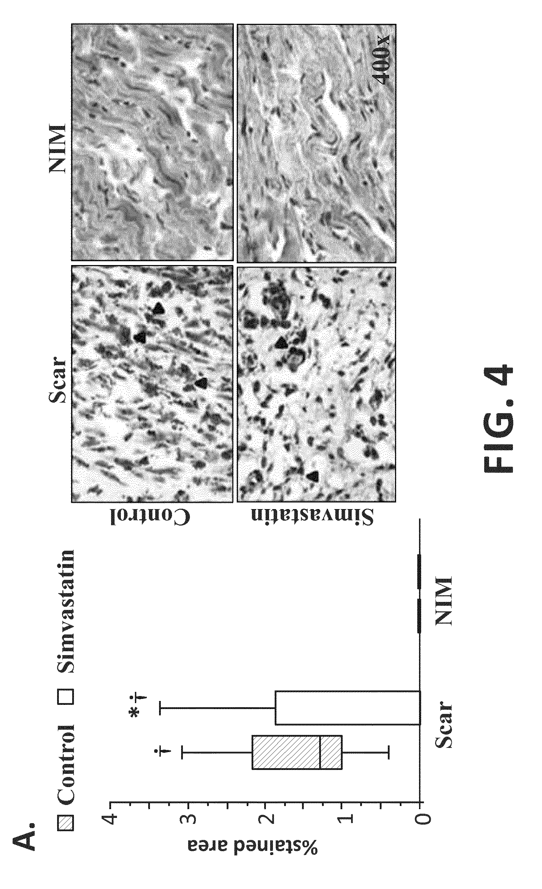

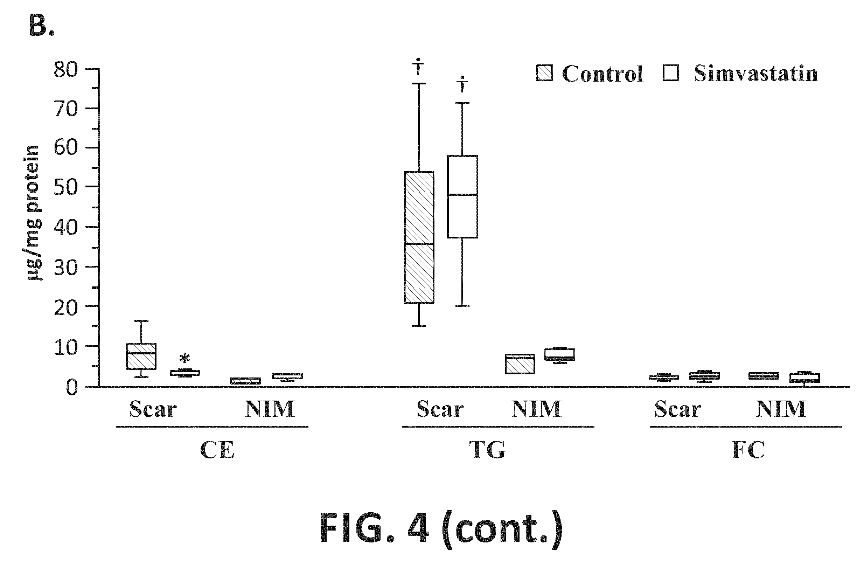

**Figure 4** shows the protective effect of simvastatin on myocardial lipotoxicity assessed 3-weeks post-MI. (A) Oil-red-O staining of the scar and remote/non-ischemic myocardium (NIM) and (B) myocardial lipid-characterization. CE: cholesterol ester; TG: triglycerides; FC: free cholesterol. *p<0.05 vs control; †p<0.05 *vs* NIM.

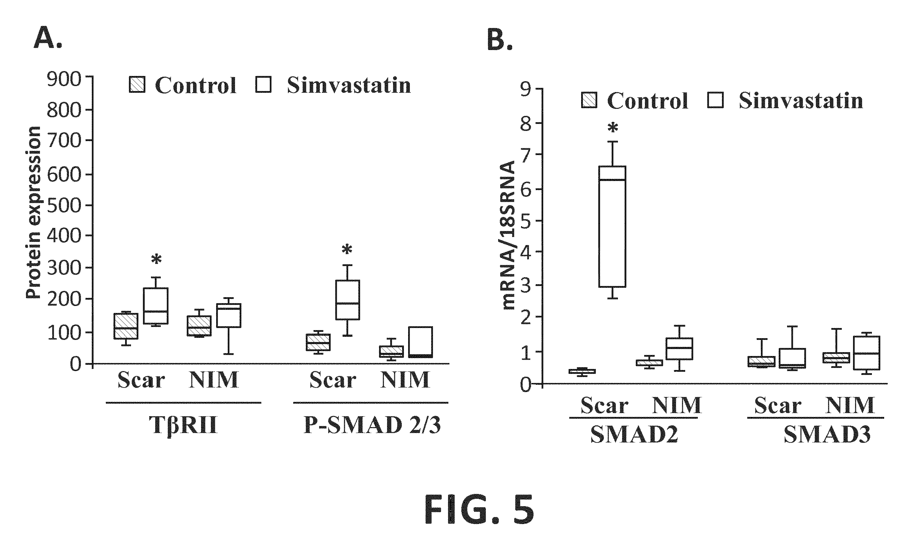

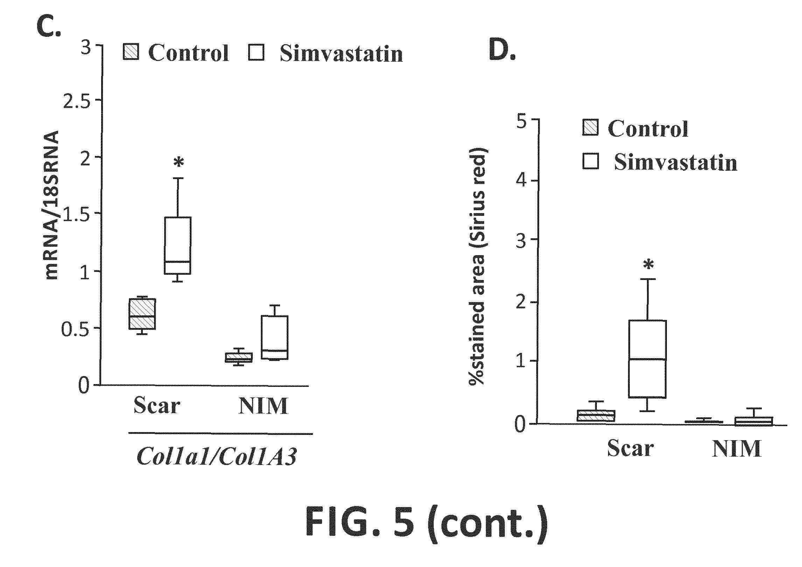

**Figure 5** shows that simvastatin favors cardiac reparative fibrosis. Sirius-red staining in control and simvastatin treated animals in the different cardiac regions. Simvastatin (A) preserves TβRII/Samd2/3 signaling pathway activation, (B) up-regulates Smad2 gene expression without altering Smad3 mRNA, (C) increases collagen mRNA expression and (D) fibril deposition (Sirius red) in the forming scar. *p<0.05 *vs* control animals.

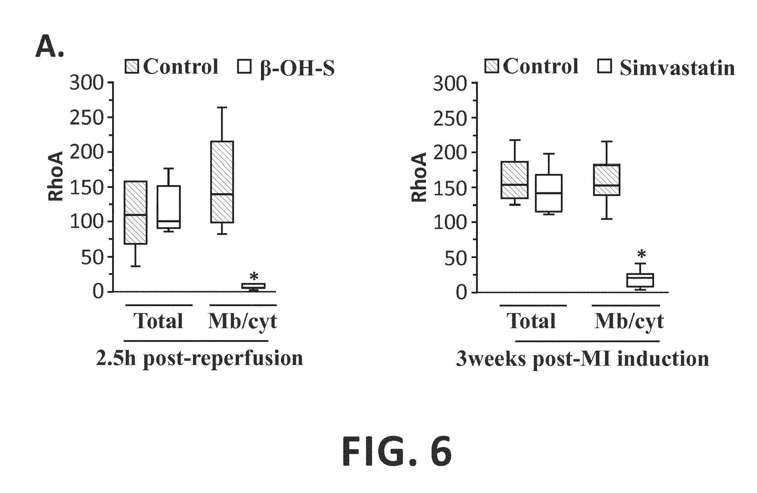

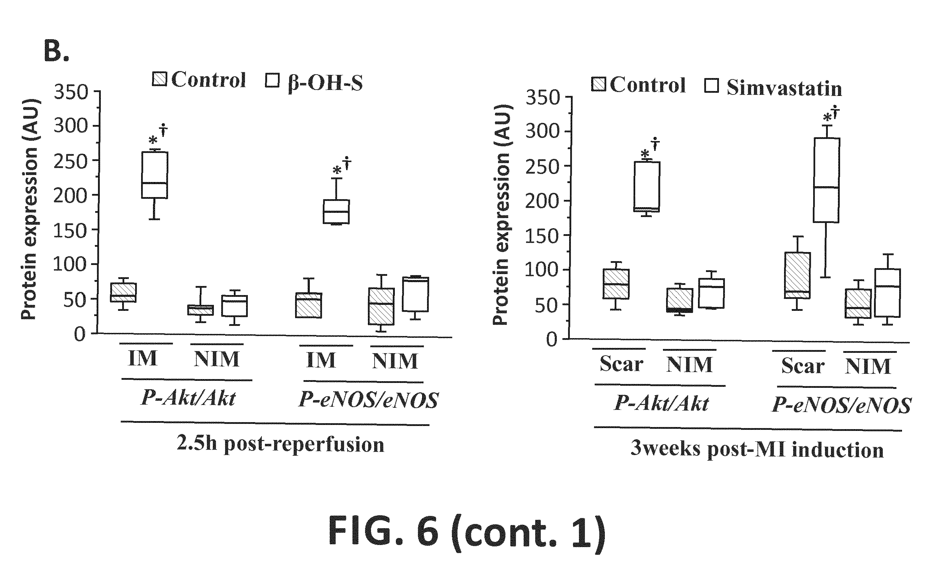

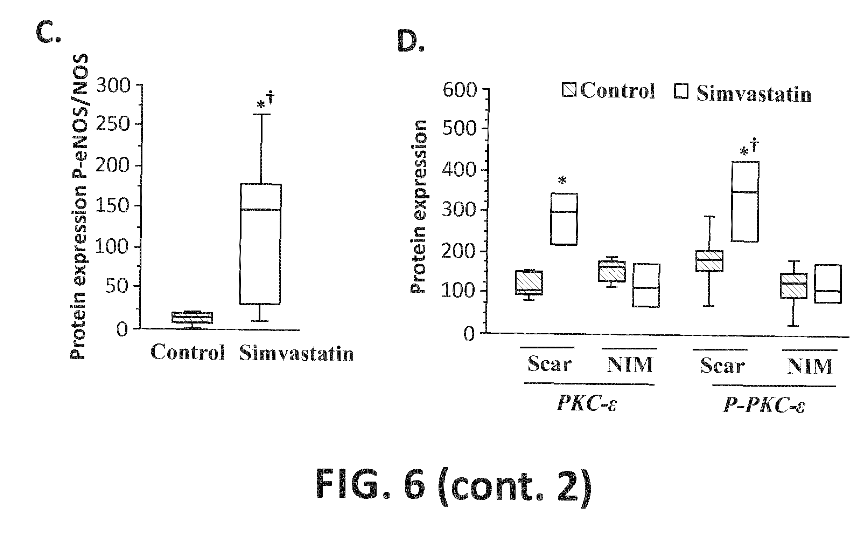

**Figure 6** shows the effect of i.v. β-OH-S administration prior to reperfusion and oral simvastatin administration post-reperfusion on myocardial RhoA and cardioprotective kinases. (A) RhoA activation in the ischemic myocardium (IM) and scar, respectively. (B) Activation of the Akt/eNOS signaling pathway. (C) Coronary eNOS activation 3-weeks post-MI. (D) ε-PKC isozyme myocardial expression/activation. *p<0.05 *vs* vehicle/control animals and †p<0.05 *vs* non-ischemic cardiac tissue.

### DETAILED DESCRIPTION OF THE INVENTION

### 1. Definitions

In order to facilitate understanding of the present patent application, the meaning of some terms and expressions in the context of the invention will be explained below.

The term "administration", as used herein, relates to the delivery of a pharmaceutical drug to a subject. Suitable routes for administration of drugs include, without limitation, the parenteral (e.g., intradermal, intramuscular, intravenous, subcutaneous, etc.), enteral (e.g., oral, rectal, etc.), topical, etc., route. In an embodiment of the invention, a statin, a statin derivative, an agent inhibiting RhoA translocation to the cell membrane, and/or an agent increasing PKCε activity is administered to the subject in need thereof, preferably intravenously, prior to reperfusion in order to prevent and/or treat ischemia/reperfusion injury. In another embodiment of the invention, a statin, a statin derivative, an agent inhibiting RhoA translocation to the cell membrane, and/or an agent increasing PKCε activity is further administered to a subject in need thereof, preferably orally, after reperfusion in order to prevent and/or treat ischemia/reperfusion injury.

The term "agent increasing PKCε activity", as used herein, relates to any compound that increases the protein kinase activity of PKCε. The term "PKCε" or "PKC epsilon", as used herein, also known as protein kinase C epsilon, relates to the epsilon member of a family of serine- and threonine-specific protein kinases that can be activated by calcium and dyacylglycerol. The term "PKCε" includes any PKCε of any subject (e.g., a mammal). Human PKCε (hPKCε) protein is encoded by the *PRKCE* gene (GeneID:5581, mRNA nucleotide sequence of 5,537 base pairs (bp) with accession number NM_005400.2 according to the NCBI nucleotide database, as of 13 April 2014). An agent increasing PKCε activity may also have, in addition to its own activity on the activity of PKCε, additional activities such as, for example, an inhibitory activity of the HMG-CoA reductase enzyme (i.e., it may function as a HMG-CoA reductase enzyme inhibitor) and/or an inhibitory activity of the translocation of the RhoA protein from cytosol to cell membrane (i.e., it may function as an inhibitor of the translocation of RhoA from to the cell membrane), among others.

The term "agent inhibiting RhoA translocation to the cell membrane", as used herein, relates to any compound that inhibits or blocks the translocation of the RhoA protein from cytosol (inactive form) to cell membrane (active). The term "RhoA", as used herein, also known as Ras homolog gene family member A, relates to a small GTPase protein that regulates the actin cytoskeleton in the formation of stress fibers. The term "RhoA" includes any RhoA of any subject (e.g., a mammal). Human RhoA protein is encoded by the *RHOA* gene (GeneID: 387, mRNA nucleotide sequence of 1,926 pb with accession number NM_001664 according to the NCBI nucleotide database, as of 27 April 2014). Similar to other GTPases, RhoA contains both inactive GDP-bound and active GTP-bound states, these states alternate between the active and inactive states via the exchange of GDP to GTP. RhoA, mostly found in the cytoplasm, is primarily involved in actin organization, cell cycle maintenance, cellular development and transcriptional control. An agent inhibiting RhoA translocation to the cell membrane may also have, in addition to its own activity on the translocation of the RhoA protein from cytosol to cell membrane, additional activities such as, for example, an inhibitory activity of the HMG-CoA reductase enzyme and/or an increasing activity of PKCε activity (i.e., it may function as an enhancer of the PKCε activity), among others.

The term "ischemia/reperfusion injury", as used herein, also known as "ischemia/reperfusion damage" relates to organ or tissue damage caused when blood supply returns to the organ or tissue after a period of ischemia. The absence of oxygen and nutrients from blood during the ischemic period creates a condition in which the restoration of circulation results in inflammation and oxidative damage through the induction of oxidative stress rather than restoration of normal function. Oxidative stress associated with reperfusion may cause damage to the affected tissues or organs. Ischemia/reperfusion injury is characterized biochemically by a depletion of oxygen during an ischemic event followed by reoxygenation and the concomitant generation of reactive oxygen species during reperfusion.

The injury that occurs with ischemia/reperfusion is the result of the interaction between the substances that accumulate during ischemia and those that are delivered on reperfusion. The cornerstone of these events is oxidative stress, defined as the imbalance between oxygen radicals and the endogenous scavenging system. The result is cell injury and death, which is initially localized, but eventually becomes systemic if the inflammatory reaction is unchecked.

In a particular embodiment, the ischemia/reperfusion injury is selected from the group consisting of organ dysfunction, infarct, inflammation, oxidative damage, mitochondrial membrane potential damage, apoptosis, reperfusion-related arrhythmia, cardiac stunning, cardiac lipotoxicity, ischemia-derived scar formation, and combinations thereof.

An ischemia/reperfusion injury can be caused by a lot of causes, for example, by a natural event (e.g., restoration of blood flow following a myocardial infarction), a trauma, or by one or more surgical procedures or other therapeutic interventions that restore blood flow to a tissue or organ that has been subjected to a diminished supply of blood. Such surgical procedures include, for example, coronary artery bypass, graft surgery, coronary angioplasty, organ transplant surgery and the like (e.g., cardiopulmonary bypass surgery). In a particular embodiment, ischemia/reperfusion injury is due to atherosclerosis, thrombosis, thromboembolism, lipid-embolism, bleeding, stent, surgery, angioplasty, end of bypass during surgery, organ transplantation, total ischemia, myocardial infarction, or combinations thereof.

The term "prevention", as used herein, relates to the capacity to prevent, minimize or hinder the onset or development of a disease or condition before its onset.

The term "reperfusion", as used herein, relates to the restoration of blood flow to the ischemic tissue. Despite the unequivocal benefit of reperfusion of blood to an ischemic tissue, it is known that reperfusion itself can elicit a cascade of adverse reactions that paradoxically injure tissue.

The term "simvastatin", as used herein, refers to (1S,3R,7S,8S,8aR)-8-{2-[(2R,4R)-4-hydroxy-6-oxotetrahydro-2H-pyran-2-yl]ethyl}-3,7-dimethyl-1,2,3,7,8,8a hexahydronaphthalen-1-yl 2,2-dimethylbutanoate, a cholesterol-lowering compound that is widely used as a potent inhibitor of the HMG-CoA reductase enzyme to treat hypercholesterolemia. The HMG-CoA reductase enzyme catalyzes the conversion of HMG-CoA to mevalonate, which is an early and rate-limiting step in the biosynthesis of cholesterol. Chemically, simvastatin is an inactive lactone that is converted to 3',5'-dihydrodiol simvastatin in liver by cytochrome P-450 (CYP) 3A after oral administration.

The term "statin", as used herein, relates to an inhibitor of the HMG-CoA reductase enzyme, which catalyzes the limiting step of cholesterol biosynthesis, e.g., the conversion of HMG-CoA to mevalonate, and includes any natural, synthetic or semi-synthetic statin. Inhibition of HMG-CoA reductase by statins decreases intracellular cholesterol biosynthesis in liver and extrahepatic tissues. Some statins can be in the closed form (lactone) or in the open form (hydroxy acid). Hydroxy acids (open form) can be prepared from the corresponding lactones by conventional hydrolysis, for example, with sodium hydroxide in methanol, sodium hydroxide in tetrahydrofuran-water and the like. In the open form (hydroxy acid), the statins react to form salts with pharmaceutically acceptable metal and amine cations formed from organic or inorganic bases. The pharmaceutically acceptable salts of the statins can differ from the corresponding free acids in some physical characteristics such as solubility and melting point. The free open form (hydroxy acid) of the statins can be regenerated from the salt form, if desired, by contacting the salt with a diluted aqueous solution of an acid such as hydrochloric acid and the like. The closed form (lactone) of the statins can be regenerated by dissolving the open form (hydroxy acid) in an inert solvent such as, for example, toluene, benzene, ethyl acetate and the like, at temperatures comprised between approximately 0°C and approximately the boiling point of the solvent, typically (although not necessarily) with simultaneous separation of the resulting water and catalysis with strong acids, e.g., hydrochloric acid and the like. A statin, in addition to its own inhibitory activity of the HMG-CoA reductase enzyme, may also have further activities such as, for example, an increasing activity of PKCε activity and/or an inhibitory activity of the translocation of the RhoA protein from cytosol to cell membrane, among others.

The term "statin derivative" relates to any molecule structurally derived from a statin, i.e., a statin having undergone a chemical derivatization such as substitution or addition of a chemical group to change (for pharmaceutical use) any of its physicochemical properties, such as solubility or bioavailability. Statin derivatives include, without limitation, statin nitroderivatives as disclosed in US 7563909 B2, and statin derivatives comprising a lactose group (which are rapidly hydrolyzed in the presence of water). In the context of the invention, statin derivatives also relate to any statin metabolite which is obtained as the result of the metabolism of a statin by a cellular enzyme, preferably resulting in an active form of the statin or statin metabolite. In a particular embodiment, the statin metabolite relates to the hydroxy acid form of a statin according to the invention. Preferably, the statin metabolite is an active form of a statin, that is to say a statin form exhibiting at least 40%, at least 50%, at least 60%, at least 70%, at least 80%, at least 90% or at least 95% of the HMG-CoA reductase activity of the statin from which is obtained. As the skilled person acknowledges, statins are usually inactive in their lactone form. Generally, the active form thereof is their metabolite statin-β-hydroxy acid formed by hydrolysis by carboxyesterase activity in both plasma/liver and intestinal mucosa. In particular, the cytochrome P450 enzyme (CYP3A4) is involved in the metabolism of most statins. Particularly preferred statin derivatives in the context of the invention are disclosed below (Table 1). A statin derivative may have at least an activity selected from the group consisting of an inhibitory activity of the HMG-CoA reductase enzyme, an increasing activity of PKCε activity, an inhibitory activity of the translocation of the RhoA protein from cytosol to cell membrane, and combinations thereof. In an embodiment, the statin derivative has an inhibitory activity of the HMG-CoA reductase enzyme. In another embodiment, the statin derivative may have at least an activity selected from the group consisting of an increasing activity of PKCε activity, an inhibitory activity of the translocation of the RhoA protein from cytosol to cell membrane, and combinations thereof. In a particular embodiment, the statin derivative may have, in addition to an inhibitory activity of the HMG-CoA reductase enzyme, further activities such as, for example, an increasing activity of PKCε activity and/or an inhibitory activity of the translocation of the RhoA protein from cytosol to cell membrane, among others.

The term "subject" or "individual" or "animal" or "patient" or "mammal", is meant any subject, particularly a mammalian subject, for whom therapy is desired. Mammalian subjects include humans, domestic animals, farm animals, and zoo, sports, or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human.

The term "treatment", as used herein, refers to both therapeutic measures and prophylactic or preventive measures, wherein the object is to prevent or slow down (lessen) an undesired physiological change or disorder, such as ischemia/reperfusion injury. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

### 2. Use of a statin or a derivative thereof, an inhibitor of RhoA translocation to the cell membrane and/or a PKCε activator in the prevention and/or treatment of ischemia/reperfusion injury

In an aspect, the present invention relates to a compound selected from the group consisting of:
a) a statin or a derivative thereof,
b) an agent inhibiting RhoA translocation to the cell membrane,
c) an agent increasing PKCε activity, and
d) any combination of a)-c),
for use in the prevention and/or treatment of ischemia/reperfusion injury in a subject, wherein said compound is administered to said subject prior to reperfusion.

Alternatively, the invention relates to the use of a compound selected from the group consisting of:
a) a statin or a derivative thereof,
b) an agent inhibiting RhoA translocation to the cell membrane,
c) an agent increasing PKCε activity, and
d) any combination of a)-c),
for the manufacture of a medicament for the prevention and/or treatment of ischemia/reperfusion injury in a subject, wherein said compound is administered to the subject prior to reperfusion.

Thus, according to the invention, the compound for use in the prevention and/or treatment of ischemia/reperfusion injury in a subject is selected from the group consisting of a statin, a statin derivative, an agent inhibiting RhoA translocation to the cell membrane, an agent increasing PKCε activity, and combinations thereof.

### 2.1 Statins and derivatives thereof

In a particular embodiment, the invention relates to a statin or a statin derivative for use in the prevention and/or treatment of ischemia/reperfusion injury in a subject.

Statins are compounds inhibiting the HMG-CoA reductase enzyme, which is the enzyme catalyzing the conversion of HMG-CoA to mevalonate. Assays to determine the HMG-CoA reductase activity of a compound are known by the skilled person and include, without limitation, those described by Liu (Liu L et al. 2003 J Pharm Biomed Anal 32(1): 107-123), Fang (Fang W et al. 2002 J Clin Lab Anal 16(5): 209-215), or Mozzicafreddo (Mozzicafreddo M et al. 2010 J Lipid Res 51(8): 2460-2463), as well as by means of kits commercially available by, without limitation, Sigma-Aldrich (Catalog No. CS1090). As it is well-known, a statin, in addition to its own inhibitory activity of the HMG-CoA reductase enzyme, may also have further activities such as, for example, an increasing activity of PKCε activity and/or an inhibitory activity of the translocation of the RhoA protein from cytosol to cell membrane, among others.

Statins exert mainly a function as inhibitors of the enzyme HMG-CoA reductase. A first group of statins comprises statins with a substituted decalin-ring structure and includes lovastatin, pravastatin and simvastatin. Lovastatin, pravastatin and simvastatin are inactive lactones which must be metabolized to their active hydroxy-acid forms in order to inhibit the HMG-CoA reductase enzyme. Therefore, statins in the first group exert their function when converted to their hydroxy-acid form of the statin. A second group of statins comprises statins wherein the butyryl group as in the statins in the first group is replaced by a fluorophenyl group, which is responsible for additional polar interactions causing a tighter binding to the enzyme. This second group statins includes fluvastatin, cerivastatin, atorvastatin and rosuvastatin, which are statins in an active hydroxy-acid form.

Statins are compounds that can be obtained by the skilled person by methods known in the art including, without limitation, those described in US 20110223640 A1, US 8471045 B2, WO 2009133089 A1 and EP 1015600 A1. Statins are also commercially available from a number of suppliers including, without limitation, Pfizer and Bayer.

Statins for use according to the invention comprise, without limitation, atorvastatin (Lipitor®, Torvast®), cerivastatin (Lipobay®, Baycol®), fluvastatin (Lescol®), lovastatin (Mecavor®, Altocor®, Altoprev®), mevastatin (Compactin®), pitavastatin (Livalo®, Pitava®), pravastatin (Pravachol®, Selektine®, Lipostat®), rosuvastatin (Crestor®) and simvastatin (Zocor®, Lipex®). In a particular embodiment, the statin for use according to the invention is simvastatin.

The pharmacokinetic properties of the statins are orchestrated by several factors, including their active or lactone form, their lipophilic/hydrophilic rate, and their absorption and metabolism. The statin pharmacological properties, including doses administered as open acid and lactone forms, are known by the skilled person and shown, for example, in Table 1 as in Gazerro P et al. 2012 Pharmacol Rev 64(1): 102-146.

Particularly preferred statins and derivatives thereof for use in the prevention and/or treatment of ischemia/reperfusion in a subject according to the invention are shown in Table 1.

**Table 1**

| **Preferred statins and statin derivatives for use in the invention** | |
|---|---|
| **Statin and formula thereof** | **Statin derivatives** |
| Atorvastatin 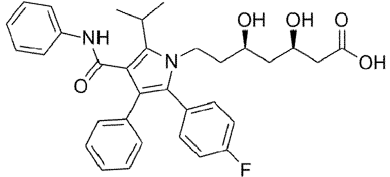 | Beta-oxidized atorvastatin, unsaturated beta-oxidized atorvastatin, beta-oxidized hydroxy atorvastatin, 4-hydroxy atorvastatin para-hydroxy atorvastatin acid, atorvastatin lactone, atorvastatin acyl glucuronide, 2-hydroxy atorvastatin ortho-hydroxy atorvastatin acid, 4-hydroxy atorvastatin acyl glucuronide, 4-hydroxy atorvastatin lactone and 2-hydroxy atorvastatin lactone. |
| Cerivastatin | Cerivastatin 6-O-glucuronide, cerivastatin 3-O-glucuronide, cerivastatin 4-O-glucuronide, M22, cerivastatin lactone, m5, m1, m4, m10, m9, m11, m20, m17, m32, m29, m21, m30, m16, m15, m18, m19, m26, m23, m28, m24 and m25. |
| 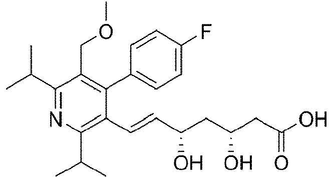 | |
| Fluvastatin | 5-hydroxy fluvastatin, 6-hydroxy fluvastatin and N-deisopropyl fluvastatin. |
| 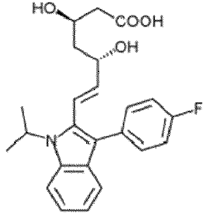 | |
| Lovastatin | Lovastatin acid, 3'-hydroxylovastatin, 6'-exomethylenelovastatin and 6'-beta-hydroxylovastatin and 3"β-hydroxylovastatin. |
| 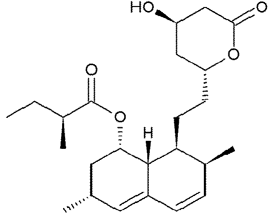 | |
| Mevastatin 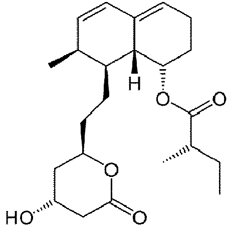 | |
| Pitavastatin | Pitavastatin lactone, 3-keto pitavastatin, pitavastatin keto lactone, m1, m18, m12, m9, hydroxy pitavastatin lactone, 3-dehydroxy pitavastatin O-glucuronide, 5-keto pitavastatin acyl glucuronide, 5-keto pitavastatin O-glucuronide and 3-dehydroxy pitavastatin acyl glucuronide. |
| 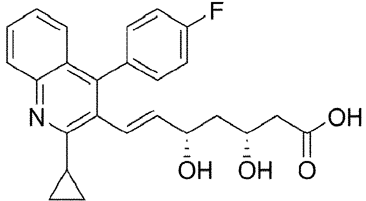 | |
| Pravastatin | 3 "(S)-hydroxytetranorpravastatin, 3'-alpha-5',6'-epoxyisopravastatin, 3'-alpha-7-hydroxyisopravastatin, 3'-ketopravastatin-5,6-diol, 3"(S)-hydroxypravastatin, 3'-alpha-isopravastatin, 3'-alpha-5'-beta,6'-beta-trihydroxypravastatin, 6'-epipravastatin, pravastatin glucuronide, desecylpravastatin, 4'-alpha-5'-beta-epoxypravastatin, pravastatin 4a'alpha-glutathione conjugate, pravastatin lactone, pravastatin sulfate, M9 and M8. |
| 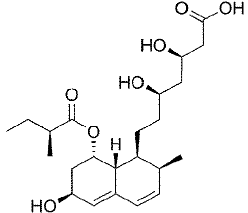 | |
| Rosuvastatin | Rosuvastatin lactone, N-demethylrosuvastatin and rosuvastatin acyl glucuronide. |
| 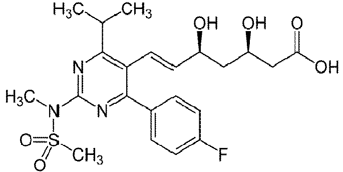 | |
| Simvastatin | 3',5'-dihydrodiolsimvastatin, 6'-beta-hydroxymethylsimvastatin, 6'beta-carboxysimvastatin, 6'-beta-hydroxysimvastatin, 6'-exomethylenesimvastatin, simvastatin acid terivastatin, simvastatin acid glucuronide and 3'-hydroxysimvastatin |
| 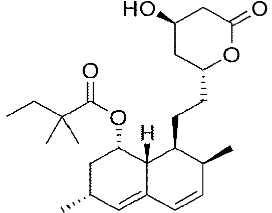 | |

In a particular embodiment, the statin or derivative thereof for use according to the present invention in the prevention and/or treatment of ischemia/reperfusion injury in a subject is a compound of formula (I), or a compound of formula (II), wherein
- the compound of formula (I) is: 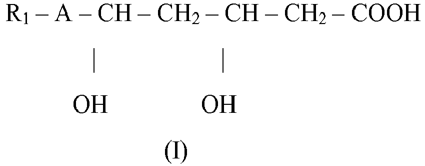 wherein
   R₁ is selected from the group consisting of 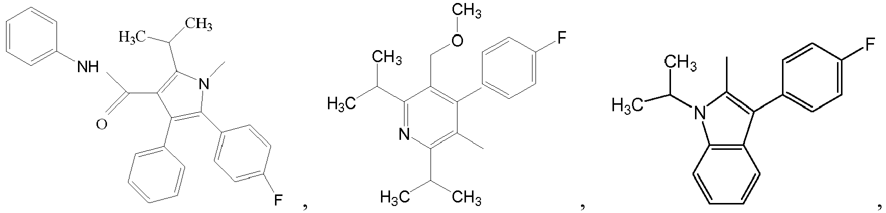 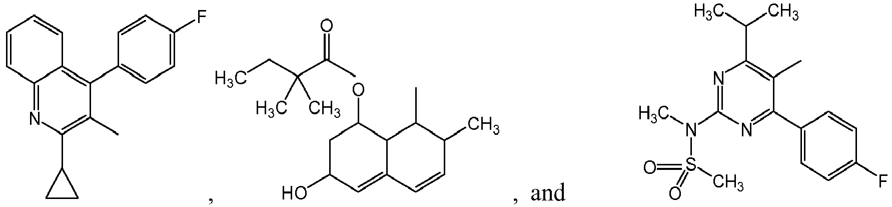 and
   A is -CH₂-CH₂- or -CH=CH-,
   or a solvate, prodrug, or pharmaceutically acceptable salt thereof, and wherein
- the compound of formula (II) is: 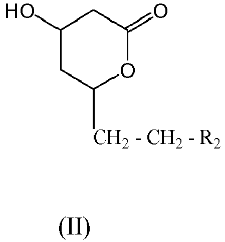 wherein R₂ is selected from the group consisting of 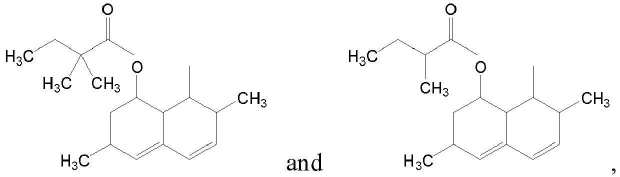
or a solvate, prodrug, or pharmaceutically acceptable salt thereof.

The term "solvate", as used herein, relates to any form of a compound of formula (I) or (II) in which said compound has attached to it via non-covalent binding other molecule (e.g., a polar solvent). Methods of solvation are known within the art.

The term "prodrug", as used herein, relates to a compound that is converted *in vivo* to any of the compounds of formula (I) or (II). Examples of prodrugs include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger "Medicinal Chemistry and Drug Discovery 6th ed. (Donald J. Abraham ed., 2001, Wiley), "Design and Applications of Prodrugs" (H. Bundgaard ed., 1985, Harwood Academic Publishers) and Krogsgaard-Larsen et al. "Textbook of Drug design and Discovery" Taylor & Francis (April 2002).

The term "pharmaceutically acceptable salt" or "physiologically acceptable salt" relates to a salt formed either with a physiologically tolerated acid, that is to say salts of a compound with inorganic or organic acids which are physiologically tolerated - especially if used on mammals, preferably in human beings - or with at least one, preferably inorganic, cation which are physiologically tolerated - especially if used on mammals, preferably in human beings. Thus, a pharmaceutically acceptable salt is a molecular entity that is physiologically tolerable and that normally does not cause an allergic reaction or similar adverse reaction, such as gastric discomfort, dizziness and the like, when they are administered to a mammal, preferably a human being. Preferably, as used in this description, the term "pharmaceutically acceptable" means approved by a governmental regulatory agency or listed in the US pharmacopoeia or another generally recognized pharmacopoeia for use in mammals, and more particularly in humans. Illustrative, non-limitative examples of pharmaceutically acceptable salts of specific acids include salts of hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid, picric acid and/or aspartic acid. Examples of physiologically tolerated salts of particular bases are salts of alkali metals and alkaline earth metals and with NH₄.

Solvates, salts and prodrugs of the compounds of formula (I) or (II) can be prepared by methods known in the state of the art.

Compounds are "stereoisomers" when they are formed by the same atoms bound by the same sequence of bonds, but with different three-dimensional structures which are not interchangeable, such as for example, enantiomers or diastereoisomers.

As the skilled person in the art can identify, the compounds of formula (I) or (II) comprise at least one asymmetric center and can therefore give rise to enantiomers with the spatial configuration (R) or (S). All the individual enantiomers of said compounds as well as their mixtures, e.g., their racemic or non-racemic mixtures, are included within the scope of the present invention. Said individual enantiomers can be separated by means of conventional techniques. Although the invention can take place using mixtures of enantiomers of said compounds of formula (I) or (II), for example, their racemic mixtures, in practice it is preferred to obtain a single enantiomer of formula (I) or (II). Likewise the compounds of formula (I) or (II) can have more than one asymmetric center and can therefore give rise to diastereoisomers. All the individual diastereoisomers of said compounds as well as their mixtures are included within the scope of the present invention. The individual diastereoisomers can be separated by means of conventional techniques.

The compound of formula (I) or (II) may have at least an activity selected from the group consisting of an inhibitory activity of the HMG-CoA reductase enzyme, an increasing activity of PKCε activity, an inhibitory activity of the translocation of the RhoA protein from cytosol to cell membrane, and combinations thereof. In an embodiment, the compound of formula (I) or (II) has an inhibitory activity of the HMG-CoA reductase enzyme. In another embodiment, the compound of formula (I) or (II) may have at least an activity selected from the group consisting of an increasing activity of PKCε activity, an inhibitory activity of the translocation of the RhoA protein from cytosol to cell membrane, and combinations thereof. In a particular embodiment, the compound of formula (I) or (II) may have, in addition to an inhibitory activity of the HMG-CoA reductase enzyme, further activities such as, for example, an increasing activity of PKCε activity and/or an inhibitory activity of the translocation of the RhoA protein from cytosol to cell membrane, among others.

Thus, in a particular embodiment of the invention the compound for use in the prevention and/or treatment of ischemia/reperfusion injury in a subject according to the invention, wherein said compound is administered prior to reperfusion, is a compound of formula (I) or (II) as previously described. Further, in another particular embodiment of the invention, the compound for use in the manufacture of a medicament for the prevention and/or treatment of ischemia/reperfusion injury in a subject according to the invention, wherein said compound is administered prior to reperfusion, is a compound of formula (I) or (II) as previously described.

In a particular embodiment of the invention, the statin or derivative thereof for use according to the present invention in the prevention and/or treatment of ischemia/reperfusion injury in a subject is simvastatin or a simvastatin derivative such as, for example, an oxime derivative of simvastatin showing increased aqueous solubility (Gupta AK et al. 2011 Asian J Pharmac Clin Res 5(1): 50-52), 3',5'-dihydrodiol simvastatin, 6'beta-hydroxymethyl simvastatin, 6'beta-carboxy simvastatin, 6'beta-hydroxy simvastatin, 6'-exomethylene simvastatin, simvastatin acid terivastatin, simvastatin acid glucuronide and 3'-hydroxy simvastatin. In a more particular embodiment, the simvastatin derivative is a beta-hydroxy derivative of simvastatin of formula (III): 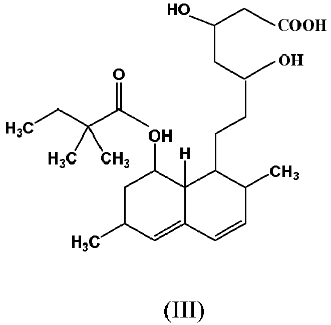 In particular, the simvastatin derivative is a beta-hydroxy derivative of simvastatin is of formula (IIIa)

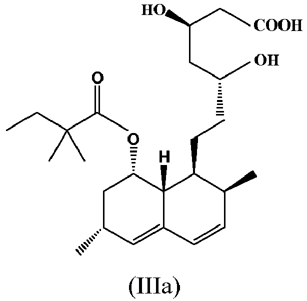

The compound of formula (III) or (IIIa) may have at least an activity selected from the group consisting of an inhibitory activity of the HMG-CoA reductase enzyme, an increasing activity of PKCε activity, an inhibitory activity of the translocation of the RhoA protein from cytosol to cell membrane, and combinations thereof In an embodiment, the compound of formula (III) or (IIIa) has an inhibitory activity of the HMG-CoA reductase enzyme. In another embodiment, the compound of formula (III) or (IIIa) may have at least an activity selected from the group consisting of an increasing activity of PKCε activity, an inhibitory activity of the translocation of the RhoA protein from cytosol to cell membrane, and combinations thereof. In a particular embodiment, the compound of formula (III) or (IIIa) may have, in addition to an inhibitory activity of the HMG-CoA reductase enzyme, further activities such as, for example, an increasing activity of PKCε activity and/or an inhibitory activity of the translocation of the RhoA protein from cytosol to cell membrane, among others.

### 2.2 RhoA inhibitors

The invention also relates to an agent inhibiting RhoA protein translocation to the cell membrane for use in the prevention and/or treatment of ischemia/reperfusion injury in a subject.

Molecular approaches to measure cytosol-membrane translocation of RhoA, which is a sign of RhoA activation, have been described in the art by Keller (Keller J et al. 1997 FEBS Lett 403(3): 299-302). Determination of total and cytosolic-membrane fractions of RhoA according to the invention has been described by some authors (e.g., Vilahur G et al. 2009 Atherosclerosis 206: 95-101).

Particularly preferred agents inhibiting RhoA translocation to the cell membrane for use in the prevention and/or treatment of ischemia/reperfusion in a subject according to the invention are shown in Table 2.

**Table 2**

| **Preferred RhoA inhibitors for use in the invention** | |
|---|---|
| **RhoA inhibitor** | **Chemical formula** |
| Tyrosine kinase inhibitor tyrphostin 23 (CAS No. 118409-57-7) | 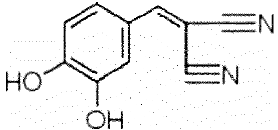 |
| Tyrosine kinase inhibitor genistein (CAS No. 446-72-0) | 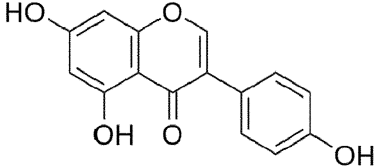 |
| Chimeric toxin DC3B, consisting of C3 and the (noncatalytic) B fragment of diphtheria toxin (Aullo P et al. 1993 EMBO J 12(3): 921-931) | |
| BA-210 (recombinant fusion protein consisting of *Clostridium botulinum* C3 exozyme-like protein lacking N-terminal 19 amino acids fused to a proline-rich peptidic transport sequence) (Cethrin) | MSAYSNTYQEFTNIDQAKAWGNAQYKKYGLSKSEKEAIVSYTKSA SEINGKLRQNKGVINGFPSNLIKQVELLDKSFNKMKTPENIMLFR GDDPAYLGTEFQNTLLNSNGTINKTAFEKAKAKFLNKDRLEYGYI STSLMNVSQFAGRPIITKFKVAKGSKAGYIDPISAFAGQLEMLLP RHSTYHIDDMRLSSDGKQIIITATMMGTAINPKEFVMNPANAQGR HTPGTRL (SEQ ID NO: 1) |
| K-115 (ripasudil hydrochloride hydrate) (4-Fluoro-5-[2(S)-methylperhydro-1,4-diazepin-1-ysulfonyl]isoquinoline hydrochloride dehydrate) | 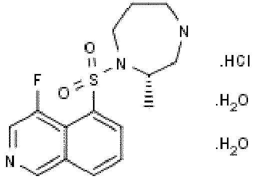 |
| Y-39983 (Senju) | 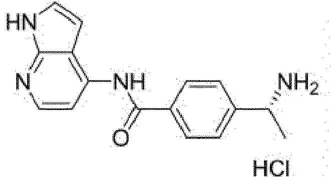 |
| DE-104 (Santen Pharmaceuticals and Ube Industries) | |
| AR-12286 (Aerie Pharmaceuticals) | 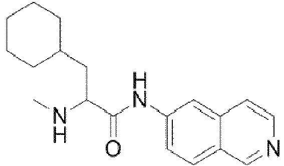 |
| AR-12286/travoprost (Aerie Pharmaceuticals) (CAS No. 157283-68-6) | 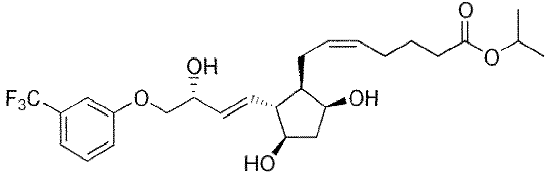 |
| AR-13324/latanoprost (Aerie Pharmaceuticals) (CAS No. 130209-82-4) | 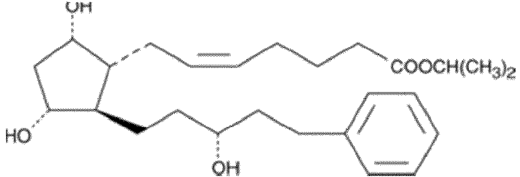 |
| ATS-907 (Altheos) | |
| AMA-0076 (Amakem) | |
| Fasudil hydrochloride (Asahi Kasei) (CAS No. 105628-07-7) | 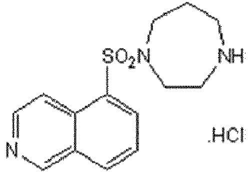 |
| SAR-407899 (Sanofi) (CAS No. 923262-96-8) | 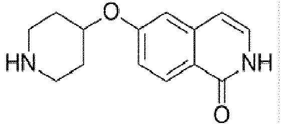 |
| *Clostridium difficile* toxin B | Accesion Number AGG91641 (2366 aa) under NCBI Protein database as of 16 March 2013 |
| *Clostridium botulinum* C3 toxin | Accesion Number 1G24_A (211 aa, chain A), 1G24_B (211 aa, chain B), 1G24_C (211 aa, chain C) and 1G24_D (211 aa, chain D) according to NCBI Protein database as of 10 October 2012. |
| A statin or derivative thereof as defined in above Section 2.1, including the compounds of Table 1 and the copounds of formula (I), (II), (III) and (IIIa) | |

In a particular embodiment, the agent inhibiting RhoA translocation to the cell membrane for use according to the invention is a RhoA-specific inhibitory antibody. The term "inhibitory antibody", as used herein, relates to an antibody which specifically binds to RhoA and is capable of inhibiting at least partially the biological activity of RhoA of translocation to the cell membrane. Methods for the obtention of antibodies are known by the skilled in the art. RhoA inhibiting antibodies include, without limitation, the rabbit polyclonal anti-RhoA (phosphor S188) antibody from Abcam (Catalog No. ab41435).

In a particular embodiment, the agent inhibiting RhoA translocation to the cell membrane for use according to the invention is a RhoA-specific RNA interference (RNAi), which is capable of knocking down the expression of RhoA gene or of any gene necessary for RhoA translocation function. In the context of the invention, the RNAi specific for RhoA may be comprised by a stable viral vector system, such as an adenovirus. Illustrative, non-limitative, examples of RhoA inhibiting RNAi for use according to the invention include, without limitation, the commercially available human RhoA RNAi from Novus Biologicals (Catalog No. H00000387-R02).

In a particular embodiment, the agent inhibiting RhoA translocation to the cell membrane for use according to the invention is a RhoA-specific short-interfering RNA (siRNA). Illustrative, non-limitative, examples of RhoA inhibiting siRNA for use according to the invention include, without limitation, human RhoA siRNA from Life Technologies (Catalog No. AM16708) and human RhoA siRNA from Santa Cruz Biotechnology (Catalog No. sc-29471).

In a particular embodiment, the agent inhibiting RhoA translocation to the cell membrane for use according to the invention is a RhoA-specific ribozyme. Ribozymes are enzymatic RNA molecules capable of catalyzing the specific cleavage of RNA. The mechanism of ribozyme action involves sequence specific hybridization of the ribozyme molecule to complementary target RNA, followed by an endonucleolytic cleavage event. The composition of ribozyme molecules preferably includes one or more sequences complementary to a target mRNA, and the well-known catalytic sequence responsible for mRNA cleavage or a functionally equivalent sequence.

In a particular embodiment, the agent inhibiting RhoA translocation to the cell membrane for use according to the invention is a RhoA-specific antisense nucleic acid, which inhibits transcription and/or translation of RhoA nucleic acid. RhoA inhibiting antisense nucleic acids according to the invention include, without limitation, human RhoA antisense compounds as disclosed in US5945290 A, and the RhoA antisense oligodeoxynucleotide 5'-TCCTGATGGCAGCCAT-3' (SEQ ID NO: 2) as described by Torsoni (Torsoni A et al. 2005 Amer J Physiol 289(1): H1488-H1496).

In a particular embodiment, the agent inhibiting RhoA translocation to the cell membrane for use according to the invention is a RhoA-specific DNA enzyme. DNA enzymes incorporate some of the mechanistic features of both antisense and ribozyme technologies. DNA enzymes are designed so that they recognize a particular target nucleic acid sequence, much like an antisense oligonucleotide, however much like a ribozyme they are catalytic and specifically cleave the target nucleic acid.

As the skilled person acknowledges, agents inhibiting RhoA translocation to the cell membrane for use according to the invention may also function as inhibitors ofHMG-CoA reductase enzyme and/or activators of PKCε activity. Thus, in a particular embodiment, agents inhibiting RhoA translocation to the cell membrane for use according to the invention exert at least an additional function selected from the group consisting of inhibition of HMCo-A reductase enzyme, and activation of PKCε activity.

### 2.3 PKCε activators

The invention also relates to an agents increasing PKCε activity for use in the prevention and/or treatment of ischemia/reperfusion injury in a subject.

Molecular approaches to determine protein kinase activity, in particular PKCε, are known by the skilled person in the art and include, without limitation, assays using radioactively labeled ATP, two-dimensional protein electrophoresis, and western blot including antibodies that specifically recognize the phosphorylated protein.

Particularly preferred agents increasing PKCε activity for use in the prevention and/or treatment of ischemia/reperfusion in a subject according to the invention are shown in Table 3.

**Table 3**

| **Preferred PKCε activators for use in the invention** | |
|---|---|
| **PKCε inhibitor** | **Chemical formula** |
| HO/03/03 (HealOr) | |
| AB-002 (a recombinant antithrombotic and cytoprotective enzyme, Aronora) | |
| Ingenol mebutate (ingenol-3-angelate, Leo Pharma) | 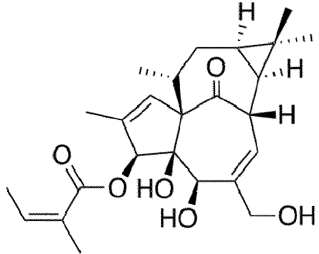 |
| 7-hydroxystaurosporine (UCN-01) (CAS No. 112953-11-4) | 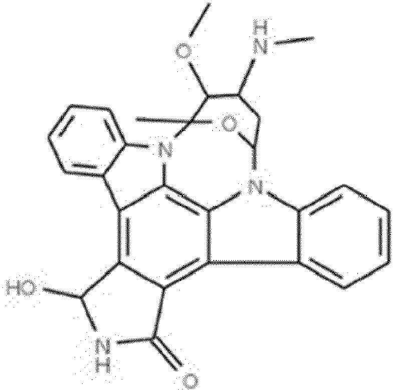 |
| MT-477 (dimethyl 5,6-dihydro-7-methoxy-5,5-dimethyl-6-(2-(2,5-dioxopyrrolidin-1-yl)acetyl)-1H-1-(4,5-dimethoxycarbonyl-1,3-dithiolo-2-spiro)thiopyrano[2,3]quinoline-2,3-dicarboxylate; Medisyn Technologies) (CAS No. 328069-91-6) | 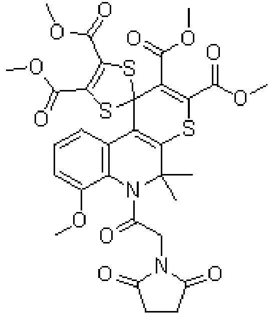 |
| Bryostatin 1 (CAS No. 83314-01-6) | 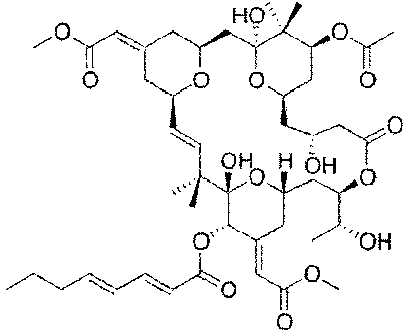 |
| Tamoxifen citrate (CAS No. 54965-24-1) | 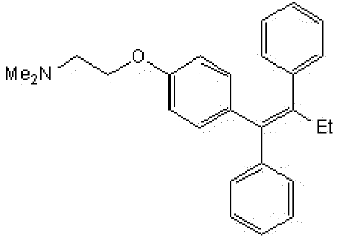 |
| Minerval® (Lipopharma) [2-Hydroxy Oleic Acid] | 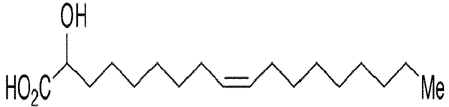 |
| Midostaurin (Novartis) (CAS No. 120685-11-2) | 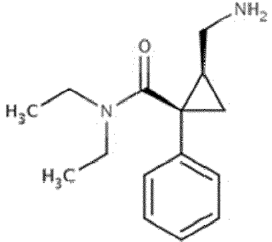 |
| Phorbol 12-myristate 13-acetate (PMA, TPA) (CAS No. 16561-29-8) | 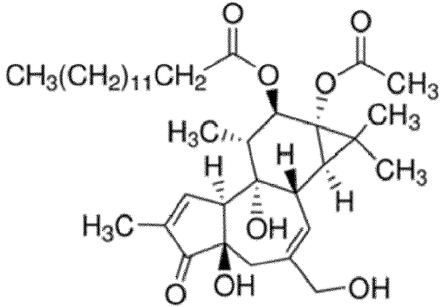 |
| AT-13148 (Astex Pharmaceuticals, Cancer Research UK) (CAS No. 1056901-62-2) | 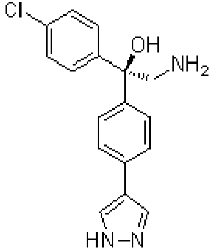 |
| 1-Stearoyl-2-arachidonoyl-sn-glycerol (CAS No. 65914-84-3) | 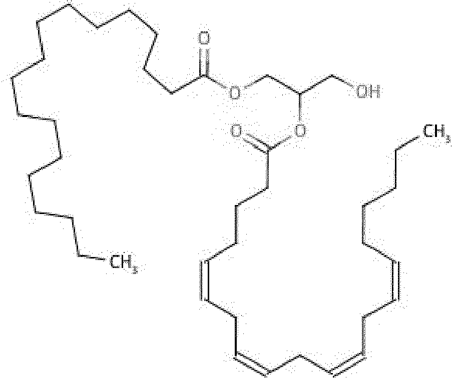 |
| Cholesterol 3-sulfate sodium salt (CAS No. 2864-50-8) | 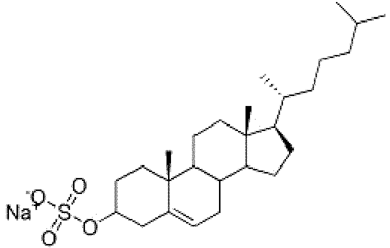 |
| Ingenol 3,20-dibenzoate (CAS No. 59086-90-7) | 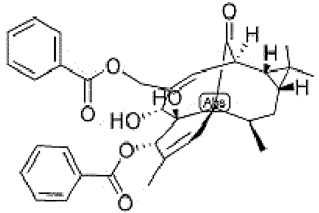 |
| Ingenol (CAS No. 30220-46-3) | 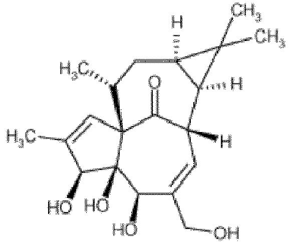 |
| Phosphatidic Acid, Dioleoyl (CAS No. 108392-02-5) | |
| L-α-Phosphatidylinositol-3,4,5-trisphosphate•7Na (PtdIns-3,4,5-P3) | |
| PI-3,4-P2, 5NH4, PIP2, Dipalmitoyl-(L-α-Phosphatidylinositol-3,4-bisphosphate, Dipalmitoyl-, Pentaammonium Salt) | |
| PI-3,4,5-P3, 7NH4, PIP3, Dipalmitoyl-(L-α-Phosphatidylinositol-3,4,5-trisphosphate, Dipalmitoyl-, Heptaammonium Salt) | |
| A statin or derivative thereof as defined in above Section 2.1, including the compounds of Table 1 and the compounds of formula (I), (II), (III) and (IIIa) | |

As the skilled person acknowledges, agents increasing PKCε activity for use according to the invention may also function as inhibitors of HMG-CoA reductase enzyme and/or inhibitors of RhoA translocation to the cell membrane. Thus, in a particular embodiment, agents increasing PKCε activity for use according to the invention exert at least an additional function selected from the group consisting of inhibition of HMCo-A reductase enzyme, and inhibition of RhoA translocation to the cell membrane.

For its administration to the subject, the compound selected from the group consisting of a statin or derivative thereof, an agent inhibiting RhoA translocation to the cell membrane, an agent increasing PKCε activity and any combination thereof, for use according to the invention, will be formulated in a pharmaceutical composition.

The term "pharmaceutical composition", as used herein, refers to a composition comprising a therapeutically effective amount of a compound selected from the group consisting of a statin or derivative thereof, an agent inhibiting RhoA translocation to the cell membrane, an agent increasing PKCε activity and any combination thereof, for use according to the invention, and at least a pharmaceutically acceptable excipient or carrier.

The term "therapeutically effective amount", as used herein in relation to the compound for use according to the invention (i.e., a statin or derivative thereof, an agent inhibiting RhoA translocation to the cell membrane, an agent increasing PKCε activity or any combination thereof), relates to an amount of said compound that provides the desired effect, for example, an appreciable prevention, cure, delay, reduction of severity or amelioration of one or more symptoms derived from ischemia/reperfusion injury. The therapeutically effective amount of a compound will be generally determined by taking into consideration different features such, for example, the characteristics of the product itself and the therapeutic effect to be achieved, the particulars of the subject to be treated, the severity of the injury suffered by said subject, the chosen dosage form, etc. For this reason, the doses mentioned herein should be considered only as guides for the person skilled in the art, who must adjust the doses depending on the aforementioned variables. In an embodiment, the therapeutically effective amount of the compound is an amount that ameliorates, attenuates or eliminates one or more symptoms of ischemia/reperfusion injury in the treated subject.

Even though individual needs vary, determination of optimal ranges for therapeutically effective amounts of the compounds for use according to the invention belongs to the common experience of those experts in the art. In general, the dosage needed to provide an effective treatment, which can be adjusted by one expert in the art, will vary depending on age, health, fitness, sex, diet, weight, degree of alteration of the receptor, frequency of treatment, nature and condition of the injury, nature and extent of impairment or illness, medical condition of the subject, route of administration, pharmacological considerations such as activity, efficacy, pharmacokinetic and toxicology profile of the particular compound used, if using a system drug delivery, and if the compound is administered as part of a combination of drugs. The amount of the compound for use according to the invention that is therapeutically effective in the prevention and/or treatment of ischemia/reperfusion injury in a subject can be determined by conventional clinical techniques (see, for example, The Physician's Desk Reference, Medical Economics Company, Inc., Oradell, NJ, 1995, and Drug Facts and Comparisons, Inc., St. Louis, MO, 1993).

The dosage regimen for the compound for use according to the invention (i.e., a statin or a statin derivative, an agent inhibiting RhoA translocation to the cell membrane, or an agent increasing PKCε activity) can vary within a broad range; nevertheless, in a particular embodiment the dosage regimen for that compound ranges, usually, from 0.001 to 1 mg/kg body weight. In a particular embodiment, when the compound for use according to the invention is a statin or a derivative thereof and it is intravenously administered prior to reperfusion, the dosage regimen of said compound ranges from 0.1 to 1 mg/kg body weight, typically from 0.2 to 0.8 mg/kg body weight, preferably between 0.3 and 0.6 mg/kg body weight. In a more particular embodiment, said statin or a derivative thereof that is intravenously administered prior to reperfusion is the β-hydroxy acid form of simvastatin of formula (III) or (IIIa), and its dosage regimen ranges from 0.1 to 1 mg/kg body weight, typically from 0.2 to 0.8 mg/kg body weight, preferably between 0.3 and 0.6 mg/kg body weight.

The terms "pharmaceutically acceptable excipient" or "pharmaceutically acceptable carrier", refer to any compound or combination of compounds that is essentially non-toxic to the subject at the dosage and concentration employed, and is compatible with the other components of a pharmaceutical composition. Thus, an excipient is an inactive substance formulated alongside the active ingredient (i.e., the compound for use according to the invention such as a statin or derivative thereof, an agent inhibiting RhoA translocation to the cell membrane, an agent increasing PKCε activity or any combination thereof) of a pharmaceutical composition, for the purpose of bulking-up compositions that contain said active ingredients. Bulking up allows convenient and accurate dispensation of a drug substance when producing a dosage form. Excipients also can serve various therapeutic-enhancing purposes, such as facilitating compound (drug) absorption or solubility, or other pharmacokinetic considerations. Excipients can also be useful in the manufacturing process, to aid in the handling of the active substance concerned such as by facilitating powder flowability or non-stick properties, in addition to aiding *in vitro* stability such as prevention of denaturation over the expected shelf life. The selection of appropriate excipients depends upon the rout of administration and the dosage form, as well as the active ingredient and other factors. An excipient can be a non-toxic solid, semisolid or liquid filler, diluent, encapsulating material or formulation auxiliary of any conventional type. Illustrative, non-limitative, examples of excipients or carriers include water, salt (saline) solutions, alcohol, dextrose, vegetable oils, polyethylene glycols, gelatin, lactose, amylose, magnesium stearate, talc, surfactants, silicic acid, viscous paraffin, perfume oil, monoglycerides and diglycerides of fatty acids, fatty acid esters petroetrals, hydroxymethyl cellulose, polyvinylpyrrolidone and the like.

The compound for use according to the invention, i.e., a statin or a derivative thereof, an agent inhibiting RhoA translocation to the cell membrane, an agent increasing PKCε activity, or any combination thereof, can be administered to a subject by any suitable route of administration, for example, parenteral (e.g., intramuscular, intravenous, subcutaneous, etc.), enteral (i.e., oral, rectal, etc.), etc.

In a particular embodiment, the pharmaceutical composition containing the compound for use in the present invention is a pharmaceutical composition for parenteral administration. Thus, said pharmaceutical composition suitable for parenteral injection, include physiologically acceptable sterile aqueous or non-aqueous solutions, dispersions, suspensions or emulsions, or may comprise sterile powders for reconstitution into sterile injectable solutions or dispersions. Examples of suitable aqueous or non-aqueous excipients or carriers, diluents, solvents or vehicles include water, ethanol, polyols (propylene glycol, polyethylene glycol, glycerol, and the like), suitable mixtures thereof, triglycerides, including vegetable oils such as olive oil, or injectable organic esters such as ethyl oleate. In a particular embodiment, the pharmaceutical composition containing the compound for use in the present invention is a pharmaceutical composition for intravenous, intramuscular or subcutaneous administration. Typically, pharmaceutical compositions for intravenous, intramuscular or subcutaneous administration are solutions in sterile isotonic aqueous buffer. If necessary, the pharmaceutical composition including the compound for use according to the invention also includes a local anesthetic to ameliorate any pain at the site of the injection. Generally, the ingredients are supplied either separately or mixed together in unit dosage form, for example, as a dry lyophilized powder or water free concentrate in a hermetically sealed container such as an ampule or sachette indicating the quantity of active ingredient. Where the pharmaceutical composition is to be administered by infusion, it can be dispensed with an infusion bottle containing sterile pharmaceutical grade water or saline. Where the pharmaceutical composition is administered by injection, an ampule of sterile water for injection or saline can be provided so that the ingredients may be mixed prior to administration.

In another particular embodiment, the pharmaceutical composition containing the compound for use in the present invention is a pharmaceutical composition for oral administration.

Solid dosage forms for oral administration include conventional capsules, sustained release capsules, conventional tablets, sustained-release tablets, chewable tablets, sublingual tablets, effervescent tablets, pills, suspensions, powders, granules and gels. In the solid dosage forms, the active ingredient (i.e., the compound for use according to the invention selected from the group consisting of a statin or a derivative thereof, an agent inhibiting RhoA translocation to the cell membrane, an agent increasing PKCε activity, and any combination thereof) is admixed with at least one suitable excipient or carrier, such as sodium citrate or dicalcium phosphate or (a) fillers or extenders, such as for example, starches, lactose, sucrose, mannitol, or silicic acid; (b) binders, such as for example, carboxymethylcellulose, alginates, gelatin, polyvinylpyrrolidone, sucrose, or acacia; (c) humectants, such as for example, glycerol; (d) disintegrating agents, such as for example, agar-agar, calcium carbonate, potato or tapioca starch, alginic acid, certain complex silicates, or sodium carbonate; (e) solution retarding agents, such as for example, paraffin; (f) absorption accelerators, such as for example, quaternary ammonium compounds; (g) wetting agents, such as for example, cetyl alcohol or glycerol monostearate; (h) adsorbents, such as for example, kaolin or bentonite; and/or (i) lubricants, as for example, talc, calcium stearate, magnesium stearate, solid polyethylene glycols, sodium lauryl sulfate, or mixtures thereof. In the case of capsules and tablets, the dosage forms may also comprise buffering agents. Solid formulations of a similar type may also be used as fillers in soft or hard filled gelatin capsules using excipients such as lactose or milk sugar, as well as high molecular weight polyethylene glycols, and the like. Solid dosage forms such as coated tablets, capsules and granules can be prepared with coatings or shells, such as enteric coatings and others known in the art. They may also contain opacifying agents, and can be formulated such that they release the active ingredient or ingredients in a delayed manner. Examples of embedding formulations that can be used are polymeric substances and waxes. The active ingredients can also be in micro-encapsulated form, if appropriate, with one or more of the aforementioned excipients.

Liquid dosage forms for oral administration include pharmaceutically acceptable emulsions, solutions, suspensions, syrups and elixirs containing suitable excipients or carriers used in the art. In addition to the active ingredient (i.e., the compound for use according to the invention selected from the group consisting of a statin or a derivative thereof, an agent inhibiting RhoA translocation to the cell membrane, an agent increasing PKCε activity, and any combination thereof), the liquid dosage form may contain one or more excipients or carriers commonly used in the art, such as water or other solvents, solubilizing agents and emulsifiers, such as for example, ethyl alcohol, isopropyl alcohol, ethyl carbonate, ethyl acetate, benzyl alcohol, benzyl benzoate, propylene glycol, 1,3-butylene glycol, dimethylformamide, oils, particular cottonseed oil, groundnut oil, corn germ oil, olive oil, castor oil, sesame seed oil, Miglyol^{®}, glycerol, tetrahydrofurfuryl alcohol, polyethylene glycols, fatty acid esters of sorbitan, or mixtures of these substances, and the like. In addition to said inert diluents, the formulation can also include adjuvants, such as wetting agents, emulsifying and suspending agents, sweetening agents, flavoring agents and perfuming agents. Suspensions, in addition to the active ingredient or ingredients, may contain suspending agents, as for example, ethoxylated isostearyl alcohols, polyoxyethylene sorbitol or sorbitan esters, microcrystalline cellulose, aluminum metahydroxide, bentonite, agar-agar, or tragacanth, or mixtures of these substances, and the like.

In another particular embodiment, the pharmaceutical composition containing the compound for use in the present invention is a pharmaceutical composition for topical administration. For topical administration, said pharmaceutical composition can be formulated as a cream, gel, lotion, liquid, pomade, spray solution, dispersion, solid bar, emulsion, microemulsion and the like which may be formulated according to conventional methods that use suitable excipients, such as, for example, emulsifiers, surfactants, thickening agents, coloring agents and combinations of two or more thereof.

The pharmaceutical composition comprising a compound for use according to the invention may be also administered in the form of transdermal patches or iontophoresis devices. Thus, in a specific embodiment, a compound for use according to the invention is administered as a transdermal patch, for example, in the form of sustained-release transdermal patch. Suitable transdermal patches are described in more detail in, for example, US5262165, US5948433, US6010715 and US6071531.

Several drug delivery systems are known and can be used to administer the compounds for use according to the invention, including, for example, encapsulation in liposomes, microbubbles, emulsions, microparticles, microcapsules and the like. The required dosage can be administered as a single unit or in a sustained release form.

Sustainable-release forms and appropriate materials and methods for their preparation are described in the art. In a particular embodiment, the orally administrable form of a pharmaceutical composition comprising a compound for use according to the invention is in a sustained release form that further comprises at least one coating or matrix. The coating or sustained release matrix include, without limitation, natural polymers, semisynthetic or synthetic water-insoluble, modified, waxes, fats, fatty alcohols, fatty acids, natural semisynthetic or synthetic plasticizers, or a combination of two or more of the them. Enteric coatings may be applied using conventional processes known to experts in the art.

In a particular embodiment, when the compound for use according to the invention comprises a nucleic acid (including without limitation siRNA, antisense oligonucleotides, and ribozymes), the pharmaceutical composition may be formulated as a composition intended for use in gene therapy; by way of illustration, not limitation, that pharmaceutical composition may contain a viral or nonviral vector, which comprises the suitable polynucleotide or gene construction. By way of illustration and not limitation, said vectors, may be viral, for example, based on retrovirus, adenovirus, etc., or nonviral such as ADN-liposome, ADN-polymer, ADN-polymer-liposome complexes, etc. [see "Nonviral Vectors for Gene Therapy", edited by Huang, Hung and Wagner, Academic Press (1999)]. Said vectors, which contain the corresponding polynucleotide or gene construction, may be administered directly to a subject by conventional methods. Alternatively, said vectors may be used to transform, or transfect or infect cells, for example, mammal cells, including human, *ex vivo,* which subsequently will be implanted into a human body or an animal to obtain the desired therapeutic effect. For administration to a human body or an animal, said cells will be formulated in a suitable medium that will have no adverse influence on cell viability.

Those skilled in the art are familiar with the principles and procedures discussed in widely known and available sources as Remington's Pharmaceutical Science (17th Ed., Mack Publishing Co., Easton, Pa., 1985), Goodman and Gilman's The Pharmaceutical Basis of Therapeutics (8th Ed., Pergamon Press, Elmsford, N.Y., 1990) and "Tratado de Farmacia Galénica", C. Fauli and Trillo, Luzán 5, S.A. de Ediciones, 1993 and in Remington's Pharmaceutical Sciences (A.R. Gennaro, Ed.), 20th edition, Williams & Wilkins PA, USA (2000), which are incorporated herein by reference.

The compound for use according to the invention, i.e., a statin or a derivative thereof, an agent inhibiting RhoA translocation to the cell membrane, an agent increasing PKCε activity, or any combination thereof, to be administered prior to reperfusion to a subject in order to prevent and/or treat ischemia/reperfusion injury, is administered at a period of time which can vary broadly; nevertheless, in a particular embodiment, the compound is administered at least 1 second prior to reperfusion, typically at least 15, 30 or 45 seconds prior to reperfusion, preferably at least 1 minute, 5 minutes, 10 minutes, 15 minutes, 30 minutes, 45 minutes prior to reperfusion, or even at least 1 hour, 2 hours or 3 hours prior to reperfusion or even before.

In a particular embodiment, the compound for use according to the invention is a statin or a derivative thereof, that is administered by the oral or parenteral route, preferably intravenously, at a dosage regimen ranging from 0.001 to 1 mg/kg body weight, at least 1 second prior to reperfusion, preferably at least 1, 5 or 15 minutes prior to reperfusion. In a more particular embodiment, said statin or derivative thereof is the β-hydroxy acid form of simvastatin of formula (III) or (IIIa), that is intravenously administered to a subject, its dosage regimen ranges from 0.1 to 1 mg/kg body weight, typically from 0.2 to 0.8 mg/kg body weight, preferably between 0.3 and 0.6 mg/kg body weight, and is administered at least 1 second prior to reperfusion, preferably at least 1, 5 or 15 minutes prior to reperfusion.

In another particular embodiment, the compound for use according to the invention is an agent inhibiting RhoA translocation to the cell membrane, that is administered by the oral or parenteral route, preferably intravenously, at a dosage regimen ranging from 0.001 to 1 mg/kg body weight, at least 1 second prior to reperfusion, preferably at least 1, 5 or 15 minutes prior to reperfusion.

In another particular embodiment, the compound for use according to the invention is an agent increasing PKCε activity, that is administered by the oral or parenteral route, preferably intravenously, at a dosage regimen ranging from 0.001 to 1 mg/kg body weight, at least 1 second prior to reperfusion, preferably at least 1, 5 or 15 minutes prior to reperfusion.

In another particular embodiment, the compound for use according to the invention is a combination of a statin or a derivative thereof with an agent inhibiting RhoA translocation to the cell membrane, or of a statin or a derivative thereof with an agent increasing PKCε activity, or of an agent inhibiting RhoA translocation to the cell membrane and an agent increasing PKCε activity, or of a statin or a derivative thereof with an agent inhibiting RhoA translocation to the cell membrane and with an agent increasing PKCε activity; that combination is administered prior to reperfusion to a subject by the oral or parenteral route, preferably intravenously, at a dosage regimen ranging from 0.001 to 1 mg/kg body weight, at least 1 second prior to reperfusion, preferably at least 1, 5 or 15 minutes prior to reperfusion.

The compound for use according to the invention may be administered alone of in combination with other drugs (e.g., in the form of a polypill or cocktail of drugs) provided that at least one of the drugs is a compound for use according to the invention.

The invention also contemplates, in a particular embodiment, the administration to the subject of a second compound, wherein said second compound is selected from the group consisting of a statin or a derivative thereof, an agent inhibiting RhoA translocation to the cell membrane, an agent increasing PKCε activity, and any combination thereof, wherein said second compound is administered after (post) reperfusion, for the prevention and/or treatment of ischemia/reperfusion injury.

Thus, according to the above particular embodiment, the invention provides a first compound and a second compound for use in the prevention and/or treatment of ischemia/reperfusion injury in a subject,
wherein said first compound is selected from the group consisting of:
a) a statin or a derivative thereof,
b) an agent inhibiting RhoA translocation to the cell membrane,
c) an agent increasing PKCε activity, and
d) any combination of a)-c), and
   is administered to said subject prior to reperfusion, and
wherein said second compound is selected from the group consisting of:
a) a statin or a derivative thereof,
b) an agent inhibiting RhoA translocation to the cell membrane,
c) an agent increasing PKCε activity, and
d) any combination of a)-c), and
   is administered to said subject after reperfusion.

Alternatively, according to this particular embodiment, the invention relates to the use of a first compound and a second compound in the manufacture of a medicament for the prevention and/or treatment of ischemia/reperfusion injury in a subject,
wherein said first compound is selected from the group consisting of:
a) a statin or a derivative thereof,
b) an agent inhibiting RhoA translocation to the cell membrane,
c) an agent increasing PKCε activity, and
d) any combination of a)-c), and
   is administered to said subject prior to reperfusion, and
wherein said second compound is selected from the group consisting of:
a) a statin or a derivative thereof,
b) an agent inhibiting RhoA translocation to the cell membrane,
c) an agent increasing PKCε activity, and
d) any combination of a)-c), and
   is administered to said subject after reperfusion.

The particulars of said compounds (statin or derivative thereof, agent inhibiting RhoA translocation to the cell membrane, and agent increasing PKCε activity) have been previously mentioned as well as pharmaceutical compositions comprising said compounds.

The first compound (i.e., the one that is administered prior to reperfusion) and the second compound (i.e., the one that is administered after (post) reperfusion) can be equal or different. Thus, in an embodiment, the first and second compounds are equal, i.e., both are the same statin or derivative thereof, or the same agent inhibiting RhoA translocation to the cell membrane, or the same agent increasing PKCε activity. In another embodiment, the first and second compounds are different, i.e., the first compound can be a statin and the second compound can be another statin or a statin derivative, or an agent inhibiting RhoA translocation to the cell membrane, or an agent increasing PKCε activity; alternatively, the first compound can be a statin derivative and the second compound can be another statin derivative or a statin, or an agent inhibiting RhoA translocation to the cell membrane, or an agent increasing PKCε activity; alternatively, the first compound can be an agent inhibiting RhoA translocation to the cell membrane and the second compound can be another agent inhibiting RhoA translocation to the cell membrane, or a statin or a derivative thereof, or an agent increasing PKCε activity; alternatively, the first compound can be an agent increasing PKCε activity and the second compound can be another agent increasing PKCε activity, or a statin or a derivative thereof, or an agent inhibiting RhoA translocation to the cell membrane.

In a particular embodiment, the second compound is a statin or statin derivative. Illustrative, non-limitative, examples of statins and derivatives thereof that can be used as the second compound according to the invention for use in the prevention and/or treatment of ischemia/reperfusion in a subject according to the invention are shown in Table 1. In a more particular embodiment, the statin is simvastatin.

In another particular embodiment, the second compound is an agent inhibiting RhoA translocation to the cell membrane. Illustrative, non-limitative, examples of agents inhibiting RhoA translocation to the cell membrane that can be used as the second compound according to the invention for use in the prevention and/or treatment of ischemia/reperfusion in a subject according to the invention are shown in Table 2.

In another particular embodiment, the second compound is an agent increasing PKCε activity. Illustrative, non-limitative, examples of agents increasing PKCε activity that can be used as the second compound according to the invention for use in the prevention and/or treatment of ischemia/reperfusion in a subject according to the invention are shown in Table 3.

The second compound for use according to the invention, i.e., a statin or a derivative thereof, an agent inhibiting RhoA translocation to the cell membrane, an agent increasing PKCε activity, or any combination thereof, can be administered to a subject by any suitable route of administration, for example, parenteral (e.g., intramuscular, intravenous, subcutaneous, etc.), enteral (i.e., oral, rectal, etc.), etc. In a particular embodiment, the second compound is orally administered. In another particular embodiment, the second compound is parenterally administered. In a more particular embodiment, the second compound is simvastatin and it is orally administered.

The dosage regimen of the above identified second compound for use according to the invention and administered after reperfusion will be determined by the physician and the clinical factors, as previously described. In a particular embodiment, the dosage regimen for that second compound ranges from 1 to 40 mg/day, usually from 15 to 35 mg/day, typically from 20 to 30 mg/day. In a particular embodiment, the second compound is a statin or a derivative thereof, preferably simvastatin, and it is administered via oral. Thus, in a more particular embodiment, the second compound is simvastatin, that is orally administered to a subject, at a dosage regimen ranging from 1 to 40 mg/day, usually from 15 to 35 mg/day, typically from 20 to 30 mg/day, such as, for example, about 20 mg/day.

The above identified second compound for use according to the invention, to be administered after reperfusion to a subject in order to prevent and/or treat ischemia/reperfusion injury, is administered in one or more administrations, at a period of time which can vary broadly. In a particular embodiment, the second compound is sequentially administered at different periods of time starting at a point of time; the point of time in which the second compound is administered for the first time to the subject can vary broadly; nevertheless, in a particular embodiment, the second compound is administered at a point of time that is at least 1 minute after reperfusion, for example, at least 5, 10, 15, 20, 25, 30, 35, 40, 45, 50 or 55 minutes after reperfusion, or even at least 1, 2, 3, 4, 5, 6, 7, 8, 9, 10, 11, 12, 13, 14, 15, 16, 17, 18, 19, 20, 21, 22, 23, or 24 hours after to reperfusion or even after; in a particular embodiment the second compound is administered when the subject is ready to receive the compound (physical state that can be determined by the specialist). In fact, in a particular embodiment, when the second compound to be administered post-reperfusion is orally administered, and wherein reperfusion is performed in a subject under anesthesia, oral administration requires that the subject regains tolerance to oral administration (i.e., ability to swallow, preferably, ability to swallow liquids). Once the second compound is administered for the first time to the subject, it can be subsequently administered to the subject at different periods of time, for example daily for at least 2, 3, 4, 5, 6 or 7 days, or for at least 1, 2, 3 or 4 weeks, or for at least 1, 2, 3, 4, 5, or 6 months or even more; in any case, the specialist can select the period of time and frequency of administration in view of the evolution of the subject.

In a more particular embodiment, simvastatin (as second compound) is administered orally firstly at a point of time comprised between 15 minutes and 24 hours, typically between 1 and 12 hours, post-reperfusion, and, subsequently it is daily administered during at least three weeks.

In a particular embodiment, the invention relates to a first compound and a second compound for use in the prevention and/or treatment of ischemia/reperfusion injury in a subject, wherein
- said first compound is a statin or a derivative thereof, preferably the β-hydroxy acid form of simvastatin of formula (III) or (IIIa), that is administered by the oral or parenteral route, preferably intravenously, at a dosage regimen ranging from 0.001 to 1 mg/kg body weight, at least 1 second prior to reperfusion, preferably at least 1, 5 or 15 minutes prior to reperfusion; and
- said second compound is a statin or a derivative thereof, which may be the same or different as the statin or derivative thereof (first compound), preferably simvastatin, that is administered by the oral or parenteral route, preferably orally, after reperfusion, for a period of time which comprises at least 1 week.

According to the invention, the ischemia/reperfusion injury is selected from the group comprising organ dysfunction (in the ischemic organ or in any other organ), infarct, inflammation (in the damaged organ or tissue), oxidative damage, mitochondrial membrane potential damage, apoptosis, reperfusion-related arrhythmia, cardiac stunning, cardiac lipotoxicity, ischemia-derived scar formation, and combinations thereof.

"Organ dysfunction" relates to a condition wherein a particular organ does not perform its expected function. An organ dysfunction develops into organ failure if the normal homeostasis cannot be maintained without external clinical intervention. Methods to determine organ dysfunction are known by the skilled person comprising, without limitation, monitorization and scores including sequential organ failure assessment (SOFA) score, multiple organ dysfunction (MOD) score and logistic organ dysfunction (LOD) score.

"Infarct" relates to a localized area of ischemic necrosis produced by anoxia following occlusion of the arterial supply or venous drainage of a tissue or organ. More particularly, a myocardial infarction (MI), commonly known as heart attack, is related to an event by which blood stops flowing properly to part of the heart, and the heart muscle is injured due to not receiving enough oxygen. Usually an infarction is the result of blockage of one of the coronary arteries due to an unstable buildup of white blood cells, cholesterol and fat. Important risk factors are previous cardiovascular disease, old age, tobacco smoking, high blood levels of LDL cholesterol and triglycerides, low levels of HDL cholesterol, diabetes, high blood pressure, lack of physical activity, obesity, chronic kidney disease, excessive alcohol consumption, and the use of cocaine and amphetamines. Methods to determine whether a subject has suffered from infarction are known in the art and include, without limitation, tracing electrical signals in the heart by an electrocardiogram (ECG), and testing a blood sample for substances associated with heart muscle damage, including creatine kinase (CK-MB) and troponin. ECG testing is used to differentiate between two types of myocardial infarctions based on the shape of the tracing. An ST section of the tracing higher than the baseline is called an ST elevation MI (STEMI) which usually requires more aggressive treatment. Methods to determine infarct size are known by the skilled person and include measurement of serum markers including creatine kinase (CK)-MB levels in a serum sample (Grande P et al. 1982 Circulation 65: 756-764), tissue staining with triphenyl tetrazolium chloride (Fishbein MC et al. 1981 Am Heart J 101(5): 593-600), technetium (Tc)-99m sestamibi single-photon emission computed tomography (SPECT) myocardial perfusion imaging, and magnetic resonance imaging (MRI) (Gibbons RJ et al. 2004 J Amer Coll Cardiol 44(8): 1533-1542).

"Inflammation", or "inflammatory response", relates to a set of changes occurring in a tissue that undergoes inflammation. In particular, inflammation relates to the biological response to harmful stimuli, including pathogens, damaged cells or irritants. Methods to determine inflammation are known in the art and include, without limitation, measure of erythrocyte sedimentation rate (ESR), wherein a higher ESR is indicative of inflammation, measure of C-reactive protein (CRP), wherein a higher level of CRP is indicative of inflammation, and leukocyte count (increased in inflammation).

"Oxidative damage" relates to the biomolecular damage that can be caused by direct attack of reactive species during oxygen restoration. Oxidative damage may involve lipid peroxidation, oxidative DNA damage and oxidative damage to proteins. Methods to determine lipid peroxidation include, without limitation, MDA (malondialdehyde)-TBA (thiobarbituric acid) determination by HPLC, and quantification of isoprostanes (which are specific end products of the peroxidation of polyunsaturated fatty acids) by mass spectrometry. Methods to determine DNA oxidative damage include, without limitation, measure of 8-hydroxy-2'-deoxyguanosine (8OHdG). Methods to determine oxidative damage to proteins include, without limitation, quantification of individual aminoacid oxidation products including kynurenines (from tryptophan), bityrosine (which appears to be metabolically stable and can be detected in urine), valine and leucine hydroxides, L-dihydroxyphenylalanine (L-DOPA), ortho-tyrosine, 2-oxo-histidine, glutamate semialdehyde and adipic semialdehyde, as well as the carbonyl assay (involving measurement of protein carbonyl groups).

The mitochondrial membrane potential (Δψm) relates to the membrane potential in the form of proton gradient across the mitochondrial inner membrane. Methods for evaluation of mitochondrial membrane potential damage are known by the skilled person and include the use of fluorescent probes for monitoring membrane potential including the JC1 dye (Cell Technology) and the measure of overall fluorescence at excitation and emission wavelengths allowing the quantification of green (485 nm and 535 nm) and red fluorescence (550 nm and 600 nm). Prolonged ischemia of any tissue or organ is known to induce mitochondrial membrane potential damage.

"Apoptosis" is related to a regulated network of biochemical events which lead to a selective form of cell suicide, and is characterized by readily observable morphological and biochemical phenomena, such as the fragmentation of the deoxyribonucleic acid (DNA), condensation of the chromatin, which may or may not be associated with endonuclease activity, chromosome migration, margination in cell nuclei, the formation of apoptotic bodies, mitochondrial swelling, widening of the mitochondrial cristae, opening of the mitochondrial permeability transition pores and/or dissipation of the mitochondrial proton gradient. Methods to determine cell apoptosis are known by the skilled person and include, without limitation, assays that measure DNA fragmentation (including staining of chromosomal DNA after cell permeabilization), assays that measure the activation of caspases such as caspase 3 (including protease activity assays), assays that measure caspase cleavage products (including detection of PARP and cytokeratin 18 degradation), assays that examine chromatin chromatography (including chromosomal DNA staining), assays that measure DNA strand breaks (nicks) and DNA fragmentation (staggered DNA ends) (including active labeling of cell nick translation or ISNT and active labeling of cells by end labeling or TUNEL), assays that detect phosphatidylserine on the surface of apoptotic cells (including detection of translocated membrane component), assays that measure plasma membrane damage/leakage (including trypan blue exclusion assay and propidium iodide exclusion assay). Exemplary assays include analysis of scatter's parameters of apoptotic cells by flow cytometry, analysis of DNA content by flow cytometry (including DNA staining in a fluorochrome solution such as propidium iodide), fluorochrome labelling of DNA strand breaks by terminal deoxynucleotidyl transferase or TdT-assay, analysis of annexin-V binding by flow cytometry and TUNEL assay.

The ischemia/reperfusion injury may also involve a reperfusion-related arrhythmia. "Arrhytmia", also known as cardiac dysrhythmia or irregular heartbeat, relates to a group of conditions in which the electrical activity of the heart is irregular, faster or slower, than normal. The heartbeat may be too fast (tachycardia, over 100 beats per minute) or too slow (bradycardia, less than 60 beats per minute), and may be regular or irregular. In some situations, arrhytmia may cause cardiac arrest. Arrhythmias can occur in the upper chambers of the heart (atria), or in the lower chambers of the heart (ventricles). Determination of arrhythmia is performed by the skilled in the art by means of electrocardiography (ECG).

"Cardiac stunning" involves different dysfunctional levels occurring after an episode of acute ischemia, despite blood flow is near normal or normal. After brief episodes of ischemia and reperfusion, prolonged mechanical dysfunction persists, although no histological signs of irreversible injury to cardiomyocytes exist, this phenomenon is called myocardial stunning. Stunning involves different facets: in addition to post-ischemic ventricular dysfunction myocardial (myocardial stunning), there is evidence of vascular/microvascular/endothelial injury (vascular stunning), post-ischemic metabolic dysfunction (metabolic stunning), long-lasting impairment of neurotransmission (neural/neuronal stunning), and electrophysiological alterations (electrical stunning).

"Cardiac lipotoxicity" involves a constellation of altered fatty acid metabolism, intramyocardial lipid overload and contractile dysfunction. Although it is unclear how lipids induce cardiac dysfunction, accumulation of intramyocardial triglyceride is associated with altered gene expression. Specifically, there is increased expression of the peroxisome proliferator-activated receptor α (PPARα)-regulated genes. PPARα is a nuclear receptor that, when activated by long chain fatty acids, induces the expression of proteins that increase the uptake and oxidation of fatty acids. Cardiac-specific overexpression of PPARα induces cardiac dysfunction in mice exposed to high circulating fatty acid levels. Pharmacologic activation of PPARα in the pressure-overloaded rat heart contributes to contractile dysfunction. In patients with diabetes and obesity, expression of the inflammatory cytokine tumor necrosis factor α (TNF-α) is increased in lipid-overloaded tissues and correlates positively with insulin resistance. TNF-α can directly cause contractile dysfunction in the heart and has been implicated in pathologic remodeling in heart failure. The accumulation of excess lipid within cardiomyocytes may lead to the production of toxic lipid intermediates, which can induce cell death.

"Scar" relates to any mark left on a tissue following the healing of a wound or damage. Particularly, this term related to the marks left in ischemic tissue. In the context of the invention, scar formation is derived from ischemia.

According to the present invention, ischemia/reperfusion injury to be prevented and/or treated according to the invention occurs in an organ or a tissue from a subject. Organs include, without limitation, brain, heart, kidneys, liver, large intestine, lungs, pancreas, small intestine, stomach, muscles, bladder, spleen, ovaries and testes. In a particular embodiment, the organ is selected from the group comprising heart, brain, kidney, intestine, pancreas, liver, lung, and skeletal muscle. In a more particular embodiment, the organ is heart. Tissues include, without limitation, nerve tissue, muscle tissue, skin tissue and bone tissue.

As the skilled person in the art acknowledges, there are different causes that may cause ischemia/reperfusion injury. In a particular embodiment, ischemia/reperfusion injury is due to atherosclerosis, thrombosis, thromboembolism, lipid-embolism, bleeding, stent, surgery, angioplasty, end of bypass during surgery, organ transplantation, a total ischemia, vascular compression by a tumor, myocardial infarction, and combinations thereof.

"Atherosclerosis" relates to any hardening of arteries secondary to atheroma or accumulation in the artery walls that is made up of inflammatory cells (mostly macrophage cells) and cell debris, that contain lipids. The artery wall thickens as a result of the accumulation of calcium and fatty materials such as cholesterol and triglyceride. The elasticity of the artery walls is reduced, impairing blood flow.

"Thrombosis" relates to the formation of a blood clot or thrombus inside a blood vessel, obstructing the flow of blood through the circulatory system.

"Thromboembolism" relates to the formation in a blood vessel of a clot (thrombus) that breaks loose and is carried by the blood stream to plug another vessel. The clot may plug a vessel in the lungs (pulmonary embolism), brain (stroke), gastrointestinal tract, kidneys, or leg.

"Lipid-embolism" or "fat embolism" refers to the often asymptomatic presence of fat globules in the lung parenchyma and peripheral circulation after long bone or other major trauma.

"Bleeding" relates to the process of losing blood or having blood flow, especially surgically. In particular, internal bleeding occurs when there is damage to an artery or vein allowing blood to escape the circulatory system and collect inside the body. The internal bleeding may occur within tissues, organs, or in cavities of the body.

"Stent" relates to a dispositive such as a tube inserted into a natural passage/conduit in the body to prevent or counteract a disease-induced, localized flow constriction.

By "surgery" or "surgical treatment" is meant any therapeutic procedure that involves methodical action of the hand or of the hand with an instrument, on the body of a human or other mammal, to produce a curative or remedial.

"Angioplasty" relates to a technique of mechanically widening narrowed or obstructed arteries, the latter typically being a result of atherosclerosis. An empty and collapsed balloon on a guide wire, known as a balloon catheter, is passed into the narrowed locations and then inflated to a fixed size using water pressures some 75 to 500 times normal blood pressure (6 to 20 atmospheres). The balloon forces expansion of the inner white blood cell/clot plaque deposits and the surrounding muscular wall, opening up the blood vessel for improved flow, and the balloon is then deflated and withdrawn. A stent may or may not be inserted at the time of ballooning to ensure the vessel remains open.

"Bypass surgery" relates to a class of surgeries involving rerouting a tubular body part and includes cardiopulmonary bypass, partial ileal bypass surgery, ileojunal bypass, gastric bypass and vascular bypass such as coronary artery bypass surgery. Cardiopulmonary bypass (CBP) temporarily takes over the function of the heart and lungs during surgery, maintaining the circulation of blood and the oxygen content of the body. Partial ileal bypass surgery is a surgical procedure which involves shortening the ileum to shorten the total small intestinal length. The ileojejunal bypass is a surgery designed as a remedy for morbid obesity. A vascular bypass is a surgical procedure performed for inadequate or loss of blood flow to a region of the body. In particular, coronary artery bypass surgery, also known as coronary artery bypass graft (CABG) surgery, is a surgical procedure performed to relieve angina and reduce the risk of death from coronary artery disease.

By "transplantation" is meant a surgical procedure by which a cell, tissue or organ is transferred from a donor subject to a recipient subject or from one part of the body to another in the same subject. The "donor subject" is the subject who gives blood, cells, tissues, or an organ for another subject by blood transfusion or an organ transplant. The donor subject is a human or another mammal. The "recipient subject" is the subject who receives blood, cells, tissues, or an organ from another subject by blood transfusion or an organ transplant. The recipient subject is a human or another mammal. Transplanted tissues comprise, but are not limited to, bone tissue, tendons, corneal tissue, heart valves, veins and bone marrow. Transplanted organs comprise, but are not limited to, heart, lung, liver, kidney, pancreas and intestine. The particular surgical procedure of transplantation wherein the donor subject and the recipient subject are genetically non-identical members of the same species is known as allotransplantation. Thus, the term allotransplant (also known as allograft, allogeneic transplant or homograft) is related to the transplantation of cells, tissues or organs sourced from a genetically non-identical member of the same species as the recipient. The term "allotransplantable" refers to organs or tissues that are relatively often or routinely transplanted. Examples of allotransplantable organs include heart, lung, liver, pancreas, kidney and intestine. The particular surgical procedure of transplantation wherein the donor subject and the recipient subject are members of different species is known as xenotransplantation. Thus, the term xenotransplant (also known as xenograft, xenogeneic transplant or heterograft) is related to the transplantation of cells, tissues or organs sourced from a donor to a recipient, wherein donor and recipient are members of different species.

"Total ischemia" relates to ischemia in which arterial and/or venous blood supply are occluded.

In a more particular embodiment, the ischemia/reperfusion injury is due to coronary obstruction (myocardial infarction) and further revascularization and blood re-flow.

"Myocardial infarction" (MI) involves an ischemic necrosis of part of the myocardium due to the obstruction of one or several coronary arteries or their branches. Myocardial infarction is characterized by the loss of functional cardiomyocytes, the myocardial tissue being irreversibly damaged. The myocardium, or heart muscle, suffers an infarction when advanced coronary disease exists, in a particular case this occurs when an atheromatous plaque located inside a coronary artery ulcerates or ruptures, causing an acute obstruction of that vessel.

The subject according to the invention is any subject for whom ischemia/reperfusion injury to an organ or tissue is to be treated and/or prevented. In a particular embodiment, the subject is a mammal, including humans, domestic animals, farm animals, and zoo, sports, or pet animals such as dogs, cats, guinea pigs, rabbits, rats, mice, horses, cattle, cows, and so on. In a more preferred embodiment of the invention, the subject is a human being. In a particular embodiment, the subject suffers from hypercholesterolemia. Hypercholesterolemia relates to a pathological condition characterized by the presence of high blood cholesterol levels, particularly total cholesterol and/or LDL levels. As used herein, the term total cholesterol refers to the sum of the sub-fractions of LDL (low density lipoprotein), HDL (high density lipoprotein) and VLDL (very low density lipoprotein) cholesterol. The person skilled in the art knows the reference values of the blood total cholesterol and LDL levels, which will vary depending on different circumstances such as age, gender, etc., and will know how to determine in each case if a subject suffers hypercholesterolemia.

### 3. Methods of treatment

In another aspect, the invention relates to a method for the prevention and/or treatment of ischemia/reperfusion injury in a subject in need thereof, hereinafter referred to as the "method of the invention", which comprises administering to said subject, prior to reperfusion, a therapeutically effective amount of a compound selected from the group consisting of: a) a statin or a derivative thereof, b) an agent inhibiting RhoA translocation to the cell membrane, c) an agent increasing PKCε activity, and d) any combination of a)-c).

The particulars of said compounds, pharmaceutical compositions comprising said compounds, routes of administration, administration dosages, amounts, periods of administration, prevention, treatment, ischemia/reperfusion injury, subject, administration, therapeutically effective amount, and reperfusion, have been previously mentioned and are incorporated herein by reference.

In a particular embodiment, the compound is a statin or statin derivative. Illustrative, non-limitative, examples of statins and derivatives thereof that can be used in the method of the invention are shown in Table 1. In a more particular embodiment, the statin or statin derivative is the beta-hydroxyacid derivative of simvastatin of formula (III) or (IIIa).

In another particular embodiment, the compound is an agent inhibiting RhoA translocation to the cell membrane. Illustrative, non-limitative, examples of agents inhibiting RhoA translocation to the cell membrane that can be used in the method of the invention are shown in Table 2.

In another particular embodiment, the compound is an agent increasing PKCε activity. Illustrative, non-limitative, examples of agents increasing PKCε activity that can be used in the method of the invention are shown in Table 3.

In another particular embodiment, the method of the invention further comprises the administration, after reperfusion, of a second compound is selected from the group consisting of: a) a statin or a derivative thereof, b) an agent inhibiting RhoA translocation to the cell membrane, c) an agent increasing PKCε activity, and d) any combination of a)-c).

The particulars of said second compounds, pharmaceutical compositions comprising said compounds, routes of administration, administration dosages, amounts, periods of administration, prevention, treatment, ischemia/reperfusion injury, subject, administration, therapeutically effective amount, and reperfusion, have been previously mentioned and are incorporated herein by reference.

The first and second compounds can be equal or different.

In a particular embodiment, the second compound is a statin or statin derivative. Illustrative, non-limitative, examples of statins and derivatives thereof that can be used in the method of the invention are shown in Table 1. In a more particular embodiment, the statin or statin derivative is simvastatin.

In another particular embodiment, the second compound is an agent inhibiting RhoA translocation to the cell membrane. Illustrative, non-limitative, examples of agents inhibiting RhoA translocation to the cell membrane that can be used in the method of the invention are shown in Table 2.

In another particular embodiment, the second compound is an agent increasing PKCε activity. Illustrative, non-limitative, examples of agents increasing PKCε activity that can be used in the method of the invention are shown in Table 3.

In a particular embodiment, the method of the invention for the prevention and/or treatment of ischemia/reperfusion injury in a subject comprises
- administering to a subject in need thereof, a first compound, wherein said compound is a statin or a derivative thereof, preferably the β-hydroxy acid form of simvastatin of formula (III) or (IIIa), that is administered by the oral or parenteral route, preferably intravenously, at a dosage regimen ranging from 0.001 to 1 mg/kg body weight, at least 1 second prior to reperfusion, preferably at least 1, 5 or 15 minutes prior to reperfusion; and, subsequently,
- administering to said subject a second compound, wherein said second compound is a statin or a derivative thereof, which may be the same or different as the statin or derivative thereof as the first compound, preferably simvastatin, that is administered by the oral or parenteral route, preferably orally, after reperfusion, for a period of time which comprises at least 1 week.

### 4. Kits and uses thereof

In another aspect, the invention relates to a kit, hereinafter referred to as the "kit of the invention", comprising, separately:
1) a first compound selected from the group consisting of:
   a) a statin or a derivative thereof,
   b) an agent inhibiting RhoA translocation to the cell membrane,
   c) an agent increasing PKCε activity, and
   d) any combination of a)-c), and
2) a second compound selected from the group consisting of:
   a) a statin or a derivative thereof,
   b) an agent inhibiting RhoA translocation to the cell membrane,
   c) an agent increasing PKCε activity, and
   d) any combination of a)-c).

Additionally, the kit of the invention can contain instructions for the sequential or separate use of the components in the kit. Said instructions can be in the form of printed material or in the form of an electronic support, capable of storing the instructions such that they can be read by a subject, such as electronic storage media (magnetic disks, tapes and similar), optical media (CD-ROM, DVD) and the like. Additionally or alternatively, the media can contain Internet addresses providing said instructions.

The particulars of said second compounds have been previously mentioned and are incorporated herein by reference.

The first and second compounds can be equal or different.

In a particular embodiment, the first compound is a statin or statin derivative. Illustrative, non-limitative, examples of statins and derivatives thereof that can be present in the kit of the invention are shown in Table 1. In a more particular embodiment, the statin or statin derivative is the beta-hydroxyacid derivative of simvastatin of formula (III) or (IIIa).

In another particular embodiment, the first compound is an agent inhibiting RhoA translocation to the cell membrane. Illustrative, non-limitative, examples of agents inhibiting RhoA translocation to the cell membrane that can be present in the kit of the invention are shown in Table 2.

In another particular embodiment, the first compound is an agent increasing PKCε activity. Illustrative, non-limitative, examples of agents increasing PKCε activity that can be present in the kit of the invention are shown in Table 3.

In another particular embodiment, the second compound is a statin or statin derivative. Illustrative, non-limitative, examples of statins and derivatives thereof that can be present in the kit of the invention are shown in Table 1. In a more particular embodiment, the statin or statin derivative is simvastatin.

In another particular embodiment, the second compound is an agent inhibiting RhoA translocation to the cell membrane. Illustrative, non-limitative, examples of agents inhibiting RhoA translocation to the cell membrane that can be present in the kit of the invention are shown in Table 2.

In another particular embodiment, the second compound is an agent increasing PKCε activity. Illustrative, non-limitative, examples of agents increasing PKCε activity that can be present in the kit of the invention are shown in Table 3.

In another aspect, the invention relates to the kit of the invention for use in the prevention and/or treatment of ischemia/reperfusion injury, wherein the first compound is intended to be administered prior to reperfusion and wherein the second compound is intended to be administered after reperfusion. Alternatively, the invention relates to the use of the kit of the invention in the prevention and/or treatment of ischemia/reperfusion injury, wherein the first compound is intended to be administered prior to reperfusion and wherein the second compound is intended to be administered after reperfusion.

The particulars of the kit of the invention have been previously mentioned and are incorporated herein by reference.

The invention is detailed below by means of the following examples which are merely illustrative and by no means limiting the scope of the invention.

### EXAMPLE 1

### Active simvastatin administered intravenously prior to reperfusion reduces ischemia/reperfusion injury in a subject

### 1.1 Materials & Methods

All procedures fulfilled the criteria established by the "Guide for the Care and Use of Laboratory Animals" published by the United States National Institutes of Health (NIH Publication No. 85-23, revised 1996).

### Animal model and study design

Twenty-four cross-bred commercial female swine were fed a Western-type hypercholesterolemic diet [20%-saturated fat (beef tallow), 2%-cholesterol, 1%-cholic acid]. This hyperlipidemic diet contains 24.82% proteins, 64.20% carbohydrates, 10.98% fats and 16,834.7 kJ. The inventors have already demonstrated that intake of this fat-rich diet for 10 days raises cholesterol to levels comparable to that found on dyslipidemic humans and induces endothelial dysfunction. After this 10 day diet period animals underwent a closed-chest 90 min mid-left anterior descending (LAD) coronary balloon occlusion. After 75 min of ischemia (15 min before reperfusion) animals were randomized to intravenous infusion (femoral vein) of an active β-hydroxy acid derivative of simvastatin (β-OH-S; 0.3 mg/kg; n=12) or vehicle (0.9% NaCl; n=12). At minute 90, the balloon was deflated to achieve coronary artery reperfusion. Animals were either sacrificed 2.5 h post-reperfusion (n=6 animals/group; to evaluate myocardial injury) or kept under their starting high-cholesterol diet regime plus oral simvastatin treatment (20 mg/day; simvastatin group; n=6) or placebo (control group; n=6) for the following 3 weeks and then sacrificed (to evaluate cardiac reparative fibrosis). The incidence of reperfusion-related arrhythmias during the first 2.5 h upon balloon deflation was documented in all animals. Animals had continuous ECG and hemodynamic monitoring throughout all procedures.

β-OH-S was obtained by alkaline treatment of simvastatin (Sigma). The intravenous loading dosage of 0.3 mg/kg β-OH-S and the oral statin dose was taken from clinical practice in humans. At the beginning of the experimental procedure animal weight was evenly matched between the two groups.

It should be noted that, although the presence of hyperlipidemia (a commonly found risk factor in patients) was analyzed, the study included juvenescent animals free from cardiovascular disease and co-medications (adenosine, nitroglycerine, beta-blockers, etc) that could interfere with the extent of cardiac injury and/or remodeling.

### Echocardiography

Transthoracic echocardiography assessment was performed in all animals before inducing ischemia (baseline), after 75 min of ischemia (prior β-OH-S infusion), 2.5 h post-reperfusion and 3 weeks post-MI using an echocardiographyc system Phillips iE33 equipped with a S5-1 Sector Array transducer. Left ventricle ejection fraction (LVEF) and shortening fraction (SF) were measured in the short-axis M-mode right parasternal projection in a plane below the mitral valves and perpendicular to the LV (left ventricle).

### Morphometric determination of infarct size and sample collection

2.5 h post-reperfusion Evan's Blue dye was injected in anesthetized animals through the left atrium to outline the area at risk (AAR) after which the hearts of the animals were arrested with potassium chloride and rapidly excised. Hearts were sectioned into 6 transverse slices parallel to the atrioventricular ring. Consecutive slices were alternatively collected for infarct size analysis (TTC; triphenyltetrazolium chloride staining) and molecular studies of the ischemic myocardium (IM) and non-ischemic myocardium (NIM). Scar tissue and NIM was obtained from animals 3 weeks post-MI for molecular/histological studies.

### Molecular studies

Tissue samples obtained from the IM (2.5 h post-reperfusion)/scar (3 weeks post-MI) and NIM of all animals were pulverized and homogenized in Tripure® or lysis buffer for RNA and protein isolation, respectively.

Transcriptomic analysis: It was analyzed by real-time PCR (Applied Biosystems) mRNA levels for: 1) apoptosis-related markers [extrinsic-(Fas Receptor/CD95 and caspase-8), intrinsic-(Bax, Bcl2, and P53) pathways and caspase-3]; 2) intracellular calcium handling receptors [sarcoendoplasmic reticulum Ca²⁺ATPase 2 (SERCA2) and the ryanodine receptor (RyR2)]; and 3) fibrous tissue formation (collagen, Smad2 and Smad3). The threshold cycle values were determined and normalized to the housekeeping gene 18S rRNA.

Western Blot analysis: Protein levels of: 1) apoptosis markers (truncated-caspase-3); 2) cardioprotective kinases [PKC-ε and PKC-ε phosphorylated at Ser⁷²⁹ (Santa Cruz) and Akt/PKB (Santa Cruz) and Akt phosphorylated at Ser⁴⁷³ (Cell signaling)]; 3) total eNOS and eNOS phosphorilated at Ser¹¹⁷⁷ (Cell Signaling); and 4) fibrosis [TGFP type II receptor (TPRII; Abcam) and phosphorylated-Smad2/3 (Ser^{423/425}; Santa Cruz)] were assessed.

Isolated mitochondrial extracts were incubated against Bcl-2 phosphorylated at Ser⁸⁷.

RhoA (Santa Cruz) was determined in total and cytosolic-membrane fractions of jeopardized cardiac tissue of all animals as previously described by Vilahur G 2009 (*ad supra*).

In addition, 3 weeks post-MI, the coronary arteries of all animals were isolated, cleaned of surrounding tissue and snap frozen for further analysis of coronary phosphorylated-eNOS protein expression.

Where appropriate the intensity ratio of the target band to P-actin was applied to provide the relative amounts of the target protein.

### Systemic oxidative stress and vascular/cardiac DNA-oxidative damage

Blood samples were collected at 2.5 h post-reperfusion and 3 weeks post-MI to evaluate lipoprotein resistance to oxidation. The inventors firstly assessed HDL-antioxidant activity by a method based on the ability of HDL to reverse oxidation of LDL. Results were expressed as the percentage of total oxidized LDL considering that control oxLDL is 100%. In addition, resistance of LDL against copper induced *in vitro* oxidation was also determined in EDTA-blood samples as previously described. The inventors also determined the lipid peroxide content of oxidized-LDL by assessing thiobarbituric-acid-reactive substances (TBARS).

Paraffin-embedded LAD and ischemic myocardial tissue of animals sacrificed 2.5 h post-reperfusion were cut into 5-µm-thick for 8-hydroxyguanosine staining (Abcam ab48508), a measurement indicative of oxidative stress-induced DNA-damage. In addition, optimal cutting temperature (OCT)-embedded ischemic myocardial tissue of animals sacrificed 2.5 h post-reperfusion was stained for neutrophils detection (anti-neutrophil elastase; Abcam). Staining was calculated by a single blinded observer from an average of 5-fields/animal as % of stained area. Images were captured by Nikon Eclipse 80i microscope and digitized by Retiga 1300i Fast camera.

### Mitochondrial membrane potential

Mitochondria were isolated from the ischemic myocardium of pigs subjected to 2.5 h of reperfusion as previously reported (Boehm et al. 2001 Am J Physiol Heart Circ Physiol 280: H977-983). Mitochondrial membrane potential (Δψm) was measured by flow cytometry using the ratiometric dye 5,5',6,6'-tetrachloro-1,1',3,3'-tetraethylbenzimidazol carbocyanine iodide (JC-1; Molecular Probes). Values were expressed as red fluorescence activity. Isolated mitochondria treated with valinomycin (Sigma), which decreases Δψm, were used as control.

### Characterization of the forming scar

Histological analysis was performed on the myocardium (scar and NIM) of all animals kept 3 weeks post-MI in order to analyze the effect of simvastatin treatment on the reparative fibrosis on the evolving scar. To that end, serially cut 5 µm sections from OCT-embedded samples were stained for lipids (oil red-O, ORO) and interstitial collagen (Sirius Red). Images were captured with a Nikon eclipse 80i microscope and digitalized by a Retiga-1300i. Staining was calculated by a single blinded observer from an average of 5-fields/sample as content (%)=[positive stained area/(total tissue area- vascular luminal areas)]x100 using ImageJ^{®}.

Lipid characterization (cholesteryl ester, triglycerides and free cholesterol content) was also performed in the same cardiac regions by thin layer chromatography (TLC) and also in the liver (statin target) following lipid extraction as previously reported (Vilahur G et al. 2012 Basic Res Cardiol 107: 291).

In addition, myofibroblasts detection in the scar tissue was performed by double staining with mouse monoclonal vimentin (Dako) and rabbit polyclonal alpha-SMA 1:50 (Abeam). Alexa fluor 488 donkey anti-mouse and alexa fluor 546 goat anti-rabbit were used as secondary antibodies, respectively and Hoechst for nuclear staining. Images were captured by confocal microscopy (20x).

### Haematological and biochemical follow-up

Blood samples were collected at baseline (prior diet administration), 2.5 h post-MI induction, and 3 weeks post-MI for lipids and liver parameters assessment and haematological counts.

### Statistical analysis

Because data were not normally distributed as observed by applying the Shapiro-Wilk test, a non-parametric statistical analysis was applied and results are reported as medians and interquartil range (IQR). For independent factors (comparisons between groups) Mann-Whitney analysis was performed; for repeated measurements Wilcoxon and Friedman analysis were appropriate. χ2-analyses of contingency tables were used to evaluate associations. All statistical tests conducted were two-sided and p<0.05 was considered significant. Statistical analysis was performed with StatView.

### 1.2 Results

### Animals follow-up

Weight gain throughout the study was comparable among the simvastatin and placebo/control animals (Table 4A). The lipid profile is shown in Table 4B. Baseline values were all within the pig physiological range. The high-cholesterol feeding model used led to a total-cholesterol/HDL ratio similar to human hypercholesterolemia at the moment of MI induction. Treatment with simvastatin did not induce significant changes in the plasma lipid profile. No alterations were observed in liver function-related parameters (Table 4C) and no variations were detected in haematological counts over time and across both animal groups.

**Table 4. Follow-up data of (A) weight progression, (B) lipid profile, and (C) liver parameters.*p<0.05 vs baseline. All animals are included in the measurements. MI: acute myocardial infarction.**

| (A) | Weight gain at MI induction (kg) | Weight gain at day 21 post-MI induction (kg) | |
|---|---|---|---|
| Control | 17.3 [16.9-17.9] | 32.1 [29.3-45.3] | |
| Simvastatin | 17.7 [16.7-22.4] | 36.9[32.1-45.9] | |
| | | | |

| (B) | Cholesterol (mg/dL) | | |
|---|---|---|---|
| | *Baseline* | *MI -induction* | *Sacrifice* |
| Control | 91[79-95] | 434[403-504]* | 536[440-601]* |
| Simvastatin | 85[71-102] | 375[337-400]* | 583[535-603]* |

| | LDL-cholesterol (mg/dL) | | |
|---|---|---|---|
| | *Baseline* | *MI -induction* | *Sacrifice* |
| Control | 36[32-48] | 278[273-290]* | 337[311-347]* |
| Simvastatin | 13[11-31] | 234[221-245]* | 324[193-474]* |

| | HDL-cholesterol (mg/dL) | | |
|---|---|---|---|
| | *Baseline* | *MI -induction* | *Sacrifice* |
| Control | 37[33-38] | 55[53-76] | 68[47-74]* |
| Simvastatin | 50[38-56] | 63[55-66] | 66[60-71]* |

| | Total-cholesterol/HDL | | |
|---|---|---|---|
| | *Baseline* | *MI-induction* | *Sacrifice* |
| Control | 2.3[2.3-2.6] | 7.9[5.7-8.8]* | 7.8[7.6-8.9]* |
| Simvastatin | 1.9[1.4-2.4] | 6.3[5.6-6.7]* | 8.1[7.6-8.2]* |

| | Triglycerides (mg/dL) | | |
|---|---|---|---|
| | *Baseline* | *MI -induction* | *Sacrifice* |
| Control | 42[39-77] | 23[11-31] | 48[25-103] |
| Simvastatin | 35[28-40] | 58[51-68] | 37[17-60] |
| | | | |

| (C) | GOT (IU/L) | | |
|---|---|---|---|
| | *Baseline* | *MI -induction* | *Sacrifice* |
| Control | 28[27-29] | 29[27-41] | 41 [37-46] |
| Simvastatin | 32[21-42] | 33[24-42] | 35[30-37] |

| | GPT (IU/L) | | |
|---|---|---|---|
| | *Baseline* | *MI -induction* | *Sacrifice* |
| Control | 38[38-42] | 37[31-41] | 52[36-54] |
| Simvastatin | 25[21-27] | 30[28-31] | 43[36-45] |

After 3 weeks, characterization of liver lipid extracts revealed a significant reduction in liver neutral lipids [cholesteryl esters (CE) and triglycerides (TG)] in the simvastatin-treated animals with respect to controls (CE: 2.6[2.4-2.7] *vs* 3.0[2.8-3.3] µg/mg protein; TG: 19.4[16.9-20.5] *vs* 24.2[21.7-28.9] µg/mg protein, respectively; p<0.05). No differences were observed as to free cholesterol content (1.4[1.3-1.5] vs 1.2[1.1-1.5] µg/mg protein, respectively).

### Intravenous infusion of β-OH-S prior reperfusion protects against reperfusion injury

- Infarct size limitation. The area at risk (AAR) was similar between both groups reaching 37% [32-38] % of the LV (Figure 1A). Yet, infarct size was 39.6% [38.4-41.5] % AAR in placebo/control animals and 23.5% [21.5-37.9]% AAR in β-OH-S-treated animals indicating that a single loading dose of active simvastatin prior reperfusion resulted in a 40% salvage of the LV-at-risk as compared to reperfusion alone (p<0.05).
- Neutrophil recruitment reduction: β-OH-S infusion exerted a powerful antiinflammatory affect in the ischemic damaged tissue resulting in a marked attenuation of neutrophils infiltration by almost 70% as compared to vehicle/control (p<0.05; Figure 1B).
- Oxidative damage reduction. β-OH-S infusion exerted a powerful protective effect against reperfusion-related oxidative damage to the coronary artery (Figure 1C) and the ischemic myocardial tissue (Figure 1D) as observed by the absence of DNA oxidative damage. In contrast, high β-OHdG staining was observed in vehicle-treated animals. Systemically, intravenous administration of β-OH-S enhanced HDL antioxidant potential as compared to vehicle-administered animals (Table 5A). As such, HDL isolated 2.5 h post-reperfusion displayed a 25% higher antioxidant activity against LDL oxidation relative to controls (p<0.05). As to LDL oxidative parameters, no differences were detected between both animal groups at 2.5 h post-reperfusion (Table 5B).

**Table 5. (A) HDL-antioxidant potential. oxLDL=100%. (B) Oxidation of LDL particles. *p<0.05 vs 2.5 h post-AMI (acute myocardial infarction) induction. †p<0.05 vs control. All animals are included in the data. β-OH-S: active β-hydroxy acid derivative of simvastatin.**

| **(A)** | | | *HDL antioxidant potencial (expressed as* % *of oxLDL)* | |
|---|---|---|---|---|
| 2.5 h post- | oxLDL+HDL | | 76 [72-81] | |
| reperfusion | from control plasma | | | |
| | oxLDL+HDL from ß-OH-S plasma | | 56 [55-58]† | |
| 3 weeks post-MI | oxLDL+HDL from control plasma | | 107 [102-112] | |
| | oxLDL+HDL from simvastatin plasma | | 66 [57-68]† | |
| | | | | |

| **(B)** | *2.5 h post-reperfusion* | | *3 weeks post-MI* | |
|---|---|---|---|---|
| | Control | ß-OH-S | Control | Simvastatin |
| Max CD, nmol DC/ mg prot LDL | 237 [230-257] | 210 [193-233] | 204 [186-227] | 216 [180-230] |
| Lag Time, min | 27 [22-28] | 25 [23-27] | 8 [7-15]* | 49 [47-53]*† |
| V max nmol * min (-1) * mg prot LDL (-1) | 8 [6-14] | 9 [7-11] | 5 [4-4] | 8 [6-9] |
| TBARS, nmol MDA/mg protein | 87 [75-93] | 95[91-105] | 110 [93-126]* | 65 [63-92]*† |

- Apoptosis execution reduction. β-OH-S infusion prevented the upregulation of several genes encoding for molecules downstream of both the death receptor- and mitochondrial- related apoptotic pathways (Figure 2A) in the jeopardized myocardium. In addition, β-OH-S diminished mitochondrial Bcl-2 activation (Figure 2B) and reduced truncated caspase-3 expression (execution of apoptosis; Figure 2C). Accordingly, the inventors detected low apoptotic cell counts (identified by TUNEL staining) within the ischemic myocardium of β-OH-S-treated animals as compared to vehicle/control (data not shown). No differences in any of the apoptosis-related parameters were detected in the non-ischemic myocardium showing values similar to those detected in the ischemic myocardium of β-OH-S-treated animals.
- Preservation of myocardial mitochondrial membrane potential (Δψm). Δψm was 2-times higher in mitochondria isolated from the ischemic myocardium of β-OH-S-treated animals relative to vehicle/control (p< 0.05) (Figure 2D). Interestingly, a two-by-two contingency table revealed a significant inverse association between mitochondrial membrane potential and myocardial apoptosis and/or oxidative stress and a direct correlation between TUNEL and myocardial 8-hydroxy-2'-deoxiguanosine (8-OHdG) staining in the ischemic myocardium (Figure 2E).
- Cardiac performance improvement. As detailed in Figure 3A, the incidence of reperfusion-related ventricular arrhythmias was higher in the control group (83% animals; 10 out of 12) than in the β-OH-S-treated group (25%; 3 out of 12).

As shown in Figure 3B ischemia induced a comparable deterioration in both LVEF (about 24% decrease) and SF (about 16% decrease) in all animals. Yet, both LVEF and SF were improved by 9.4% and 6.0%, respectively, in β-OH-S-treated animals 2.5 h after reperfusion, an effect that persisted up to 3 weeks post-MI in simvastatin-treated animals. In contrast, placebo animals displayed a further 5% decrease in both LVEF and SF. All animals displayed similar hemodynamic parameters (heart rate and mean blood pressure) throughout the study (average: 55[54-55] mm Hg and 74[73-75] bpm) and they were not affected by β-OH-S treatment.

Calcium regulatory proteins SERCA2 and RyR2 mRNAs transcripts were not affected by β-OH-S intravenous administration (ischemic myocardium: 1.9±0.5 *vs* 1.8±0.2 SERCA/18S rRNA and 0.14±0.03 *vs* 0.08±0.03 RyR2/18S rRNA and NIM: 1.5±0.3 *vs* 1.6±0.2 SERCA/18S rRNA 0.2±0.07 *vs* 0.11±0.03 RyR2/18S rRNA; β-OH-S *vs* control, respectively).

### Simvastatin favors reparative scar formation

- Attenuation of myocardial lipotoxicity. ORO staining evidenced significantly lower lipid infiltration in the evolving scar of simvastatin-treated animals 3 weeks post-MI induction (Figure 4A). Lipids were undetectable in the non-ischemic myocardium of all animals. Lipid characterization revealed 50% less cholesteryl-ester accumulation in the forming scar of simvastatin-treated animals as compared to placebo-controls (Figure 4B). Triglycerides were found in similar levels in the scar tissue of simvastatin and control animals and were significantly higher as compared to the NIM-area. No differences were detected in free-cholesterol content among the different group of animals across the different cardiac zones (Figure 4B).
- Preservation of myofibroblasts transdifferentation. Simvastatin treatment was associated with a marked and significant increase in TβRII protein expression and subsequent activation of its downstream effector Smad2/3 in the scar area (Figure 5A). Gene expression analysis of the transcription factors Smad2 and Smad3 revealed that Smad2 was significantly upregulated in the scar tissue of simvastatin animals whereas no changes were detected as to Smad3 mRNA levels throughout the different cardiac regions (Figure 5B). Simvastatin-related activation of the TβRII/SMAD2/3 pathway in the healing scar was accordingly associated with a higher detection of α-SMA/vimentin positive myofibroblasts (data not shown) and subsequent higher collagen1A1/collagen1A3 mRNA ratio (Figure 5C) and collagen fibrils deposition as compared to control animals and NIM (Figure 5D).
- Inhibition of RhoA mobilization and activation of Akt/eNOS and PKCε. Total myocardial RhoA protein expression as well as both the cytosolic (inactive) and membrane (active) fractions were determined by immunoblotting in all animals (2.5 h post-reperfusion and 3 weeks post-MI). As depicted in Figure 6A the total amount of RhoA was unchanged. However, intravenous infusion of β-OH-S markedly decreased RhoA membrane translocation (activation), measured as a prominent decrease in the membrane-to-cytosol ratio, an effect that persisted after the 3-week oral treatment (p<0.001 *vs* control; Figure 6A). In contrast to vehicle/control animals, acute β-OH-S administration was also capable of activating the P-Akt/P-eNOS pathway in the ischemic myocardium an effect that persisted in the evolving scar up to 3 weeks post-AMI (Figure 6B). Interestingly, P-eNOS was also found to be enhanced in the coronary arteries of simvastatin-fed animals at 3-weeks post-MI (Figure 6C). Finally, the inventors assessed PKCε in the forming scar, an isozyme known to participate in physiological remodeling. As shown in Figure 6D simvastatin treatment enhanced both the expression and activation of PKCε in the scar tissue as compared to placebo-control animals (Figure 6D).
- Decrease of oxidative stress. HDL antioxidant potential, already observed upon reperfusion, was maintained by daily oral simvastatin administration up to 3-weeks post-MI (Table 5A). Conversely, control animals only subjected to the Western-type diet decreased their HDL antioxidant capacity by 30% over time (76[72-81]% *vs* 107 [102-112]%; Table 3A).

A 3 week oral simvastatin treatment also lead to a significant increase in LDL resistance to *in vitro* oxidation determined as a prolongation of the lag time (almost 2 times longer *vs* acute-MI induction and 4-times longer as compared to control/placebo animals; p<0.05; Table 5B). In contrast, non-treated animals showed a higher susceptibility to LDL oxidation (lag time was shortened by 50% in compared to the experimental day). TBARS levels were also found elevated in control/placebo animals after a 3 week high fat/high cholesterol diet. In contrast, oral simvastatin treatment was associated with a 32% decrease in TBARS levels as compared to the experimental day (p<0.05) indicating a marked diminishment in the degree of lipid peroxidation. No differences were detected in the capacity of LDL to reach oxidation (maximal conjugated dienes and maximal velocity of conjugated diene formation) among both animal groups (Table 5B).

### 1.3 Discussion

The inventors have used a swine model of myocardial infarction and short-term diet-induced hypercholesterolemia. In this model, hypercholesterolemia is associated with diminished coronary vasodilation, intramyocardial lipotoxicity and the impairment of the reparative fibrotic response (TGFβ/TβRII/Smad2/3 signaling pathway) adversely affecting collagen deposition in the forming scar leading to enlarged myocardial infarcts and cardiac dysfunction. The present study demonstrates that intravenous infusion of a loading dose of activated simvastatin (β-OH-S) 15 minutes prior the restoration of the coronary flow limits myocardial injury by counteracting the inflammatory response, oxidative damage, diminishing apoptosis execution, preserving mitochondrial membrane potential and attenuating both reperfusion-related arrhythmias and cardiac stunning. Moreover, the inventors have evidenced that, as soon as 3 weeks post-MI, continuation with simvastatin oral treatment leads to a reduction in cardiac lipotoxicity (mainly reduction in intracellular cholesteryl-ester lipid accumulation) and stimulation of the TβRII/Smad2 signaling pathway and PKCε activation favoring the subsequent reparative fibrotic response in the evolving scar. Such beneficial effects are due to the direct inhibitory effect of the statin on RhoA membrane translocation allowing Akt/eNOS activation.

Firstly, the inventors demonstrated that a single infusion of β-OH-S markedly attenuated reperfusion-related inflammatory response by modulating myocardial infiltration of neutrophils, a key component of reperfusion injury, at the site of ischemia. Neutrophils not only are an important source of reactive oxygen species but largely contribute to the non-reflow phenomena thereby exacerbating tissue damage and subsequently amplifying inflammation-derived deleterious effects. The inventors also observed a powerful β-OH-S-related antioxidant effect in both the coronary artery and myocardium. Statin vascular and myocardial preservation against the overall injurious burst of oxygen-free-radicals upon reperfusion may have limited apoptotic cell death and preserved cardiac mitochondrial membrane potential. Moreover, taking into consideration that reactive oxygen species generation has shown to collapse mitochondrial Δψ inducing electrophysiological alterations in healthy hearts, the detected maintenance of mitochondrial Δψ in β-OH-S-treated animals may have largely contributed to prevent the appearance of reperfusion-related arrhythmias.

Here the inventors provide evidence that intravenous β-OH-S is capable of inducing eNOS activation during ischemia even under dyslipidemic conditions. Such eNOS preservation may favor myocardial perfusion and microvascular permeability partly attenuating cardiac dysfunction (i.e., stunning) and limiting infarct size. As such, the inventors detected a rapid recovery in heart contractibility post-MI which persisted up to 3 weeks.

The cardioprotective effects of simvastatin can be attributed to the inhibition of isoprenylation of small G-proteins rather to a reduction in serum cholesterol. The inventors observed a pronounced myocardial inhibition of RhoA translocation to the membrane (i.e., inactivation state) after a single intravenous dose of β-OH-S. RhoA inhibition by statins showed to induce the activation and translocation of Akt to the cell plasma membrane. Moreover, such Akt activation was found to be inhibited upon loading the cells with cholesterol. Interestingly, in the forming scar control animals showed a significant myocardial lipotoxicity in concurrence with Akt inactivation whereas simvastatin-treated animals displayed lower lipid infiltration (cholesteryl esters accumulation) and enhanced Akt activation. The inventors have evidenced cardiomyocyte capacity to internalize and accumulate cholesteryl-ester via LRP-1 receptor expression which, in tum, is found to be up-regulated by hypercholesterolemia. Moreover, the inventors have shown that simvastatin reduces cholesterol ester accumulation in vascular smooth muscle cells induced by aggregated LDL, an effect reversed by geranyl-geraniol (involved in RhoA activity). Therefore, it may concluded that simvastatin, via RhoA inhibition, attenuates cardiomyocyte-lipid overload favoring activation/translocation of Akt and subsequent eNOS phosphorylation. Moreover, taking into consideration that the inventors have demonstrated that myocardial-lipid infiltration is a reperfusion-related phenomenon, simvastatin may have triggered these protective effects upon reperfusion (likely reducing the extent of myocardial injury) and that continued oral statin administration may have further favored and sustained the healing phase.

Cardiac healing involves fibrous tissue formation at the site of cardiomyocyte loss in order to preserve structural integrity and requires a series of coordinated molecular and cellular events in which the TGFβ/TβRII signaling plays a critical role. TGFβ/TβRII governs fibroblast transdifferentiation into myofibroblasts and via its downstream receptor-associated effectors Smad2/3 regulates the transcription of specific extracellular matrix-related genes, primarily collagen, modulating fibrous tissue deposition. The inventors have reported that intramyocardial lipid infiltration is associated with impairment in the TGFβ/Smad2/collagen signaling pathway impeding the reparative fibrotic response post-MI. Herein, the inventors evidenced that simvastatin treatment preserves TβRII expression, Smad2/3 activation and collagen synthesis in the evolving scar helping to maintain myocardial shape and structure.

Finally, the results provided herewith also attribute to statin treatment a marked upregulation and enhanced activation of ε-protein kinase C, known to modulate cell death and cardiac remodeling. The inventors provide evidence that statins enhance and stimulate the PKC-ε isozyme over a three-week period regardless the presence of hyperlipemia.

## Claims

1. A compound selected from the group consisting of
a) a statin or a derivative thereof,
b) an agent inhibiting RhoA translocation to the cell membrane,
c) an agent increasing PKCε activity, and
d) any combination of a)-c),
for use in the treatment and/or prevention of ischemia/reperfusion injury in a subject, wherein said compound is administered to said subject prior to reperfusion.

2. The compound for use according to claim 1, wherein the compound is orally or parenterally administered to the subject prior to reperfusion, preferably intravenously.

3. The compound for use according to any of claims 1 or 2, wherein the compound is administered at least 1 second prior to reperfusion, preferably at least 1 minute prior to reperfusion, more preferably at least 15 minutes prior to reperfusion.

4. The compound for use according to any of claims 1 to 3, wherein the treatment and/or prevention of ischemia/reperfusion injury further comprises the administration to said subject of a second compound, wherein said second compound is selected from the group consisting of:
a) a statin or a derivative thereof,
b) an agent inhibiting RhoA translocation to the cell membrane,
c) an agent increasing PKCε activity, and
d) any combination of a)-c), and
wherein said second compound is administered to the subject after reperfusion.

5. The compound for use according to claim 4, wherein the second compound is orally or parenterally administered to the subject after reperfusion, preferably orally.

6. The compound for use according to any of claims 4 or 5, wherein the second compound is sequentially administered at different periods of time.

7. The compound for use according to any of claims 4 to 6, wherein the second compound is administered for the first time at a point of time that is at least 1 minute after reperfusion, preferably at least 15 minutes after reperfusion, and subsequently, daily for at least 1 day, preferably for at least 1 week, more preferably for at least 3 weeks.

8. The compound for use according to any of claims 1 to 7, wherein the compound is:
- a statin or a derivative thereof shown in Table 1, or
- a compound of formula (I) 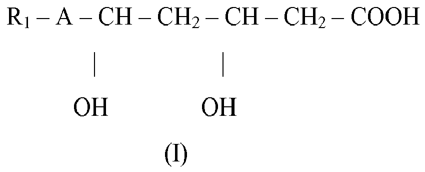 wherein
R₁ is selected from the group consisting of 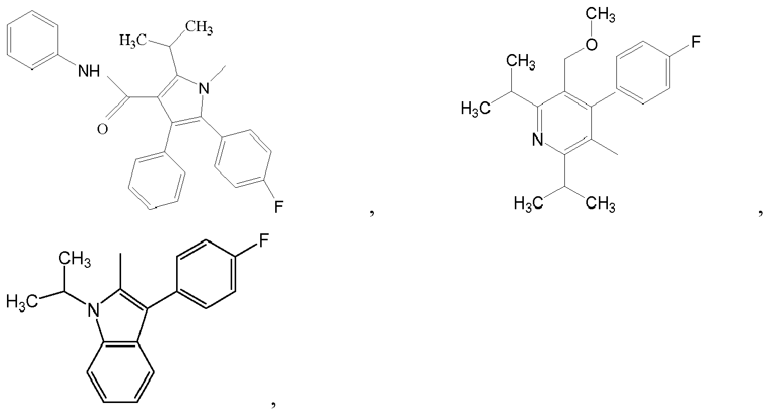 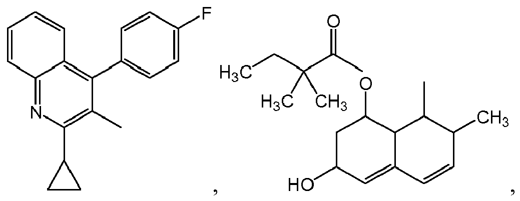 and 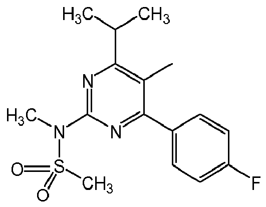 and
A is -CH₂-CH₂- or -CH=CH-,
or a solvate, prodrug, or pharmaceutically acceptable salt thereof, or
- a compound of formula (II) 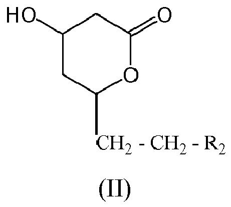 wherein R₂ is selected from the group consisting of 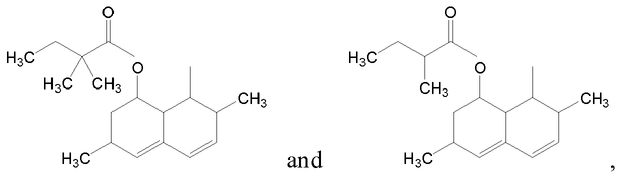 or a solvate, prodrug, or pharmaceutically acceptable salt thereof; or
- a compound of formula (III) 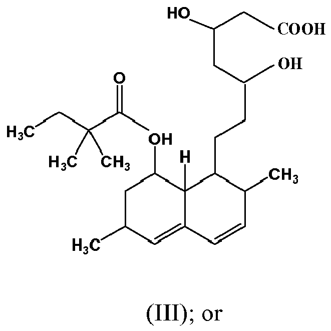
- a compound of formula (IIIa):

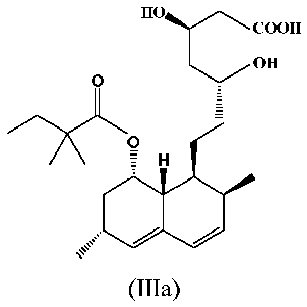

9. The compound for use according to any of claims 4 to 6, wherein the first and the second compound are, independently,
- a statin or a derivative thereof shown in Table 1, or
- a compound of formula (I) 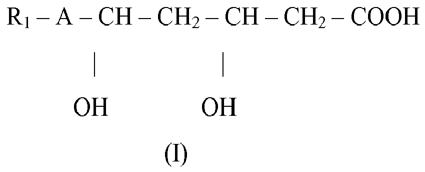 wherein
R₁ is selected from the group consisting of 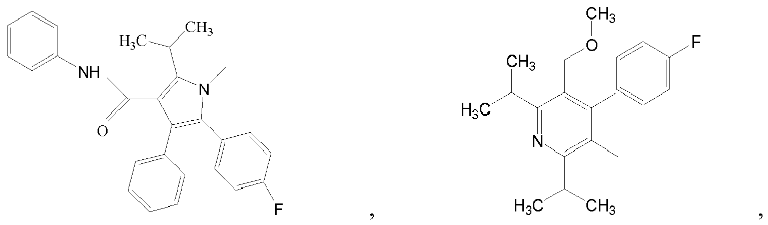 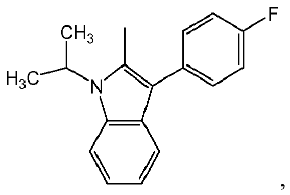 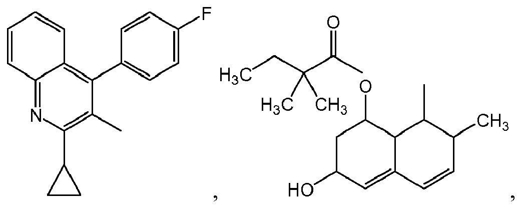 and 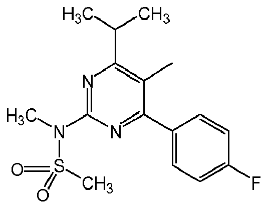 and
A is -CH₂-CH₂- or -CH=CH-,
or a solvate, prodrug, or pharmaceutically acceptable salt thereof, or
- a compound of formula (II) 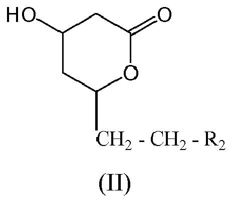 wherein R₂ is selected from the group consisting of 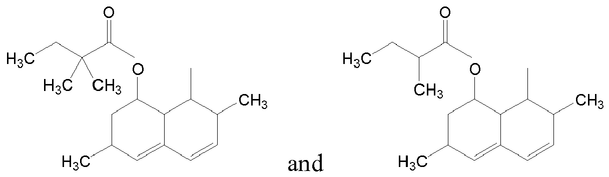 or a solvate, prodrug, or pharmaceutically acceptable salt thereof; or
- a compound of formula (III) 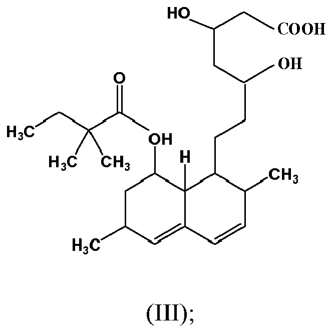 or
- a compound of formula (IIIa):

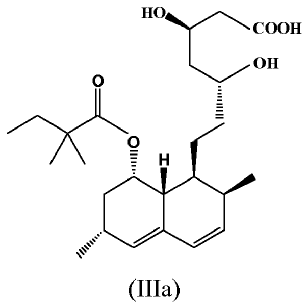

10. The compound for use according to any of claims 5 to 9, wherein said compound is a statin or a derivative thereof, preferably the β-hydroxy acid form of simvastatin of formula (III) or (IIIa), that is orally or parenterally administered, preferably intravenously, at a dosage regimen ranging from 0.001 to 1 mg/kg body weight, at least 1 second prior to reperfusion, preferably at least 1, 5 or 15 minutes prior to reperfusion; and wherein the prevention and/or treatment of ischemia/reperfusion injury in a subject further comprises a second compound wherein said second compound is a statin or a derivative thereof, preferably simvastatin, that is orally or parenterally administered to said subject, preferably orally, after reperfusion, for a period of time which comprises at least 1 week.

11. The compound for use according to any of claims 1 to 10, wherein the ischemia/reperfusion injury is selected from the group consisting of organ dysfunction, infarct, inflammation, oxidative damage, mitochondrial membrane potential damage, apoptosis, reperfusion-related arrhythmia, cardiac stunning, cardiac lipotoxicity, ischemia-derived scar formation, and combinations thereof.

12. The compound for use according to any of claims 1 to 12, wherein the ischemia/reperfusion injury is produced in an organ or a tissue selected from the group consisting of heart, brain, kidney, intestine, pancreas, liver, lung, skeletal muscle and combinations thereof, or wherein the ischemia/reperfusion injury is due to atherosclerosis, thrombosis, thromboembolism, lipid-embolism, bleeding, stent, surgery, angioplasty, end of bypass during surgery, organ transplantation, total ischemia, myocardial infarction, or combinations thereof

13. A kit comprising, separately:
1) a first compound selected from the group consisting of: a) a statin or a derivative thereof, b) an agent inhibiting RhoA translocation to the cell membrane, c) an agent increasing PKCε activity, and d) any combination of a)-c), and
2) a second compound selected from the group consisting of: a) a statin or a derivative thereof, b) an agent inhibiting RhoA translocation to the cell membrane, c) an agent increasing PKCε activity, and d) any combination of a)-c).

14. Use of a kit according to claim 13, in the prevention and/or treatment of ischemia/reperfusion injury, wherein the first compound is intended to be administered prior to reperfusion and wherein the second compound is intended to be administered after reperfusion.
